# EUROPÄISCHE PATENTSCHRIFT

(11) **EP 1 584 323 B1**
(45) Veröffentlichungstag und Bekanntmachung des Hinweises auf die Patenterteilung: **24.10.2007**
(21) Anmeldenummer: 05003964.3
(22) Anmeldetag: 24.02.2005
(51) Int. Cl.: A61Q 5/00, A61Q 5/06, A61Q 5/10, A61Q 5/12, A61K 8/73

(54) **Verwendung von N-Hydroxyalkyl-O-benzylchitosanen zur Haarbehandlung**
Use of N-hydroxyalkyl-O-benzylchitosans in hair treatment
Utilisation de N-hydroxyalcoyle-O-benzylchitosanes pour le soin des cheveux

(30) Priorität: 08.04.2004 DE 102004017431
(43) Veröffentlichungstag der Anmeldung: 12.10.2005
(73) Patentinhaber: Wella Aktiengesellschaft, 64274 Darmstadt (DE)
(72) Erfinder: Krause, Thomas, Dr., 64297 Darmstadt (DE); Baumeister, Jan, Dr., 1726 Farvagny-le-Grand (CH); Weber, Dirk, Dr., 1723 Marly (CH); Lang, Günther, Prof. Dr., 64354 Reinheim (DE); Beyer, Angelika, 63857 Waldaschaff (DE); Florig, Ellen, 64689 Grasellenbach (DE); Gänger, Klaus, 64319 Pfungstadt (DE); Schiemann, Hartmut, Dr., 36088 Hünfeld (DE)

(56) Entgegenhaltungen:
- EP-A- 0 224 045
- EP-A- 0 664 301
- DE-A1- 10 019 140
- US-A- 4 954 619

## Beschreibung

Gegenstand der vorliegenden Erfindung sind die Verwendung von N-Hydroxyalkyl-O-benzylchitosanen zur Haarbehandlung sowie Haarbehandlungsmittel mit Gehalt an N-Hydroxyalkyl-O-benzylchitosanen in Kombination mit weiteren Haarbehandlungswirkstoffen.

Ein ansprechendes äußeres Erscheinungsbild wurde schon immer als sehr wichtig angesehen. Eine besondere Rolle spielen dabei die Haare und die Frisur. Zur Verbesserung des Erscheinungsbildes werden Haarbehandlungsprodukte verwendet. Diesen Produkten ist gemeinsam, dass sie in der Regel aus einer Vielzahl an Einzelsubstanzen oder Komponenten bestehen, die die unterschiedlichsten Aufgaben innerhalb der Rezeptur erfüllen. Zu den vielseitigsten Inhaltsstoffen von kosmetischen Produkten mit den unterschiedlichsten Funktionen und Wirkungen gehören die Polymere. Polymere können beispielsweise verdickend, konservierend, filmbildend, haarfestigend, haarpflegend oder konditionierend auf Haare wirken. Sie können die Produktkonsistenz positiv beeinflussen, den Transport und die Haftung von Wirkstoffen auf dem Haar verbessern oder die Eigenschaften der übrigen Inhaltsstoffe vorteilhaft modifizieren. Es besteht ein besonderer Bedarf an Wirkstoffen, welche aufgrund von verbesserten Eigenschaften und einer besonders guten Kompatibilität mit möglichst einer Viehlzahl von anderen, üblicherweise verwendeten kosmetischen Wirk- und Zusatzstoffen möglichst durchgängig in einer ganzen Produktserie einsetzbar sind. Von besonderem Vorteil sind Polymere, die auf der Basis von nachwachsenden Rohstoffen beruhen und die gut biologisch abbaubar sind.

Es ist bereits bekannt, Chitin und Chitosane zu derivatisieren und in Haarbehandlungsmitteln einzusetzen. Hierzu zählen beispielsweise Hydroxypropylchitosane (EP 0 192 925, EP 0 224 045), Hydroxybutylchitosan (WO 87/06461), Alkylhydroxypropylchitosan (WO 86/4590), N-Hydroxypropyl-isopropyletherchitosan (WO 88/05790), N-Hydroxybutylchitosan (WO 87/6461), N-Hydroxyethylchitosan (DE 36 02 402) und Hydroxyalkylchitin (EP 0 806 435). Aus der EP 0 300 234 sind O-Benzyl-N-hydroxyalkylchitosane zur Verwendung in Nagellacken bekannt. Aus der DE 100 19 140 sind N,O-substituierte Chitin- und Chitosanderivate zur Verwendung u.a. in kosmetischen Mitteln bekannt, wobei die für Nagellacke bekannten O-Benzyl-N-hydroxyalkylchitosane explizit ausgenommen und als für (weitere) kosmetische Zubereitungen nachteilig beschrieben sind. Chitosane und Chitosanderivate werden in der Regel durch Protonierung oder Salzbildung in Wasser gelöst. Dies hat den Nachteil, dass hierdurch die Verträglichkeit mit anionischen Inhaltsstoffen wie z.B. anionischen Polymeren oder anionischen Tensiden verschlechtert wird und auch die haarpflegende und haarfestigende Wirkung abnimmt. Die bisher bekannten Chitosanderivate haben aufgrund ihrer Löslichkeitseigenschaften oder wegen Inkompatibilitäten nur beschränkte Einsatzmöglichkeiten und sind in ihren haarpflegenden Eigenschaften noch nicht ganz zufriedenstellend.

Die Aufgabe bestand darin, Wirkstoffe zur Haarbehandlung zu finden mit guten haarkosmetischen Eigenschaften und einem möglichst breiten Einsatzspektrum aufgrund von guten Löslichkeits- und Kompatibilitätseigenschaften.

Eine solche Klasse von Wirkstoffen wurde mit den unten näher definierten N-Hydroxyalkyl-O-benzylchitosanen gefunden. Diese sind in einer Vielzahl von verschiedenen haarkosmetischen Mitteln vorteilhaft einsetzbar und mit einer Vielzahl von weiteren haarkosmetischen Wirk- und Zusatzstoffen vorteilhaft kombinierbar.

Gegenstand der Erfindung ist daher die Verwendung mindestens eines N-Hydroxyalkyl-O-benzylchitosans zur Haarbehandlung. N-Hydroxyalkyl-O-benzylchitosane sind Polymere, welche abgeleitet sind von Chitosan welches mindestens eine Aminogruppe aufweist, die mit mindestens einer Hydroxyalkyl- oder Poly-(hydroxyalkyl)gruppe substituiert ist welches mindestens eine Benzylethergruppe aufweist. Die Hydroxyalkyleinheit weist eine oder mehrere Hydroxygruppen und vorzugsweise 2 bis 20 C-Atome aufweist. Besonders bevorzugt sind C2- bis C4-Alkylgruppen mit einer Hydroxygruppe, z.B. Hydroxyethyl, Hydroxypropyl und Hydroxybutyl, insbesondere Hydroxypropyl. Es können auch Gemische von N-Hydroxyalkyl-O-benzylchitosanen mit Hydroxyalkylresten unterschiedlicher Kettenlänge eingesetzt werden. Auch kann ein und dasselbe N-Hydroxyalkyl-O-benzylchitosan mit Hydroxyalkylresten unterschiedlicher Kettenlänge substituiert sein.

Geeignete N-Hydroxyalkyl-O-benzylchitosane sind erhältlich aus Chitosan durch N-Hydroxyalkylierung mit einem Alkylenoxid und gleichzeitiger oder anschließender O-Benzylierung mit einer reaktiven Benzylverbindung. Eine geeignete Synthesemethode ist in der EP 0 300 234 beschrieben. Durch Reaktion von Chitosan mit mindestens einem Alkylenoxid, z.B. Ethylenoxid, Propylenoxid und/oder Butylenoxid, vorzugsweise Propylenoxid in Abwesenheit eines basischen Katalysators ist N-Hydroxyalkylchitosan erhältlich (N-Alkoxylierung). Durch Umsetzen des N-Hydroxyalkylchitosans mit mindestens einer reaktiven Benzylverbindung, z.B. Benzylhalogeniden, vorzugsweise Benzylchlorid, bildet sich N-Hydroxyalkyl-O-benzylchitosan (O-Benzylierung). Die Herstellung ist auch, wie in der EP 0 300 234 beschreiben, als Einstufen-Verfahren durchführbar. Beim dem zugrundeliegende Chitosan kann es sich um ein vollständig oder partiell deacetyliertes Chitin handeln. Zur Herstellung von Chitosan geht man vorzugsweise von dem in den Schalenresten von Krustentieren enthaltenem Chitin aus, welches als billiger und natürlicher Rohstoff in großen Mengen zur Verfügung steht. Geeignete Chitosane sind kommerziell erhältlich. Das Molekulargewicht des Chitosans kann über ein breites Spektrum verteilt sein, z.B. von 20.000 bis ca. 5 Millionen, von 30.000 bis 1.000.000, vorzugsweise von 100.000 bis 800.000 g/mol. Der Deacetylierungsgrad beträgt z.B. 10 bis 99%, 60 bis 95%, vorzugsweise 70 bis 90%.

Geeignet sind z.B. N-Hydroxyalkyl-O-benzylchitosane, welche mindestens eine Einheit der allgemeinen Formel 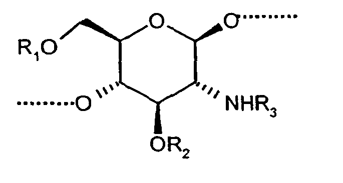 aufweisen, wobei R¹ und R² unabhängig voneinander eine Gruppe -(A-O)ₙ-H bedeuten, A eine C2- bis C20-, vorzugsweise C2- bis C4-Alkylengruppe, ist, n eine Zahl größer gleich Null ist und R³ für Wasserstoff, -CH₂Ph oder -CO-CH₃ steht, mit der Maßgabe, dass bei mindestens einer Einheit n größer Null und R³ gleich -CH₂Ph sind und/oder dass bei mindestens einer ersten Einheit n größer Null und R³ gleich Wasserstoff oder -CO-CH₃ und bei mindestens einer zweiten Einheit n gleich Null und R³ gleich -CH₂Ph sind.

Der Hydroxyalkylierungsgrad liegt z.B. im Bereich von 0,1 bis 10, vorzugsweise größer 1 bis 8, insbesondere von 1,5 bis 6. Der Acetylierungsgrad liegt vorzugsweise im Bereich von 0 bis 0,6. Der Benzylierunsgrad liegt vorzugweise im Bereich von 0,1 bis 4, vorzugsweise 0,2 bis 2. Der Polymerisationsgrad liegt vorzugsweise im Bereich von 50 bis 5.000. Das Molekulargewicht liegt vorzugsweise im Bereich von 5.000 bis 2.000.000, insbesondere im Bereich von 20.000 bis 1.000.000 oder 300.000 bis 700.000 g/mol. Geeignete N-Hydroxyalkyl-O-benzylchitosane sind insbesondere solche, die hergestellt sind aus Chitosan durch Umsetzung mit 2 bis 7 Äquivalenten Alkylenoxid (vorzugsweise Propylenoxid) und 0,25 bis 1,5 Äquivalenten Benzylchlorid pro Glucosamineinheit.

Gegenstand der Erfindung sind auch Haarbehandlungsmittel mit einem Gehalt an
(A) mindestens einem ober näher beschriebenen N-Hydroxyalkyl-O-benzylchitosan und
(B) mindestens einem weiteren Haarbehandlungswirkstoff.

Haarbehandlungswirkstoffe sind z.B. haarpflegende, haarschützende, haarreparierende, haarreinigende, haarfärbende, haarfestigende, den Haarglanz erhöhende oder die Haarform verändernde, d.h Dauerwell- oder Haarglättungswirkstoffe. Die Wirkstoffe sind vorzugsweise ausgewählt aus haarpflegenden Tensiden, insbesondere kationischen oder kationaktiven Tensiden, haareinigenden Tensiden, haarpflegenden Ölen und Wachsen, haarpflegenden Polymeren, haarpflegenden Silikonverbindungen, haarfestigenden Polymeren, Lichtschutzstoffen, Oxidationshaarfarbstoffvorprodukten, direktziehenden Haarfarbstoffen, haarfärbenden Pigmenten, Oxidationsmitteln und Keratin reduzierenden Stoffen.

Das N-Hydroxyalkyl-O-benzylchitosan (A) ist in dem erfindungsgemäßen Mittel vorzugsweise in einer Menge von 0,01 bis 20, besonders bevorzugt von 0,1 bis 10, ganz besonders bevorzugt von 0,2 bis 5 Gew.% und der Wirkstoff (B) in einer Menge von 0,01 bis 20, besonders bevorzugt von 0,05 bis 10, ganz besonders bevorzugt von 0,1 bis 5 Gew.% enthalten.

Bei den erfindungsgemäßen Haarbehandlungsmitteln kann es sich um Mittel für die Reinigung und Pflege des Haares wie z.B. Shampoos, Haarkuren, Haarspülungen, Spitzenfluids, Haaröle, Brillantine, die in den unterschiedlichsten Anwendungsarten, z.B. als leave on- oder als rinse off-Produkte, appliziert werden können; um permanente, semipermanente oder temporäre Haarfärbemittel, z.B. oxidative Haarfärbemittel oder nicht-oxdidative Haartönungsmittel oder um Haarbleichmittel; um permanente Haarverformungsmittel, z.B. in Form von mildalkalischen oder sauren Dauerwellmitteln, Haarentkrausungsmitteln oder Dauerwellfixiermitteln; sowie um Mittel für die temporäre Verformung und/oder Stabilisierung der Frisur (Stylingmittel), z.B. Haarsprays, Haarlacke, Festigerlotionen, Festigerschäume, Haargele, Haarwachse, Frisurcremes etc.handeln.

Die bisher zur Haarbehandlung eingesetzten Chitosanderivate ließen sich nur schlecht in Haarpfleformulierungen einarbeiten. Setzt man demgegenüber erfindungsgemäß N-Hydroxyalkyl-O-benzylchitosane in typischen Haarpflegemitteln, z.B. in Pflegeschäumen und Shampoos ein, so erhält man stabile Formulierungen. Die N-Hydroxyalkyl-O-benzylchitosane zeigen eine gute Spreitwirkung auf dem Haar. Erfindungsgemäß behandeltes Haar weist einen verbesserten Volumeneffekt und im nassen und trockenen Zustand einen guten Griff und gute Kämmbarkeit auf. Auch bei einem Einsatz in typischen Haarstylingmitteln wie z.B. Haarsprays erhält man stabile Formulierungen. Insbesondere eine Kombination von N-Hydroxyalkyl-O-benzylchitosanen mit haarfestigenden Polymeren führt neben einer Haarfestigung zu einer Verbesserung von Glanz und Griff der behandelten Haare.

In einer Ausführungsform enthält das erfindungsmäße Mittel als haarpflegenden oder haarfestigenden Wirkstoff mindestens ein Polymer mit anionischen oder anionisierbaren Gruppen in einer Menge von vorzugsweise 0,01 bis 20 Gew.% oder von 0,05 bis 10 Gew.%, besonders bevorzugt von 0,1 bis 5 Gew.%. Unter anionisierbaren Gruppen werden Säuregruppen wie z.B. Carbonsäure-, Sulfonsäure- oder Phosphorsäuregruppen verstanden, welche mittels üblicher Basen wie z.B. organischer Amine oder Alkali- oder Erdalkalihydroxide deprotoniert werden können.

Die Polymere der Komponente (B) können teilweise oder vollständig mit einem basischen Neutralisationsmittel neutralisiert sein. Bevorzugt sind solche Mittel, in welchen im Polymer der Komponente (B) die sauren Gruppen zu 50 bis 100 %, besonders bevorzugt zu 70-100% neutralisiert sind. Als Neutralisationsmittel können organische oder anorganische Basen verwendet werden. Beispiele für Basen sind insbesondere Aminoalkanole wie z.B. Aminomethylpropanol (AMP), Triethanolamin oder Monoethanolamin, aber auch Ammoniak, NaOH, KOH u.a..

Das Polymer (B) kann ein Homo- oder Copolymer mit Säuregruppen enthaltenden Monomereinheiten auf natürlicher oder synthetischer Basis sein, welches gegebenenfalls mit Comonomeren, die keine Säuregruppen enthalten, copolymerisiert ist. Als Säuregruppen kommen Sulfonsäure-, Phosphorsäure- und Carbonsäuregruppen in Betracht, von denen die Carbonsäuregruppen bevorzugt sind. Geeignete Säuregruppen enthaltende Monomere sind beispielsweise Acrylsäure, Methacrylsäure, Crotonsäure, Maleinsäure bzw. Maleinsäureanhydrid, Maleinsäuremonoester, insbesondere die Mono-C1-C7-alkylester der Maleinsäure sowie Aldehydocarbonsäuren oder Ketocarbonsäuren. Nicht mit Säuregruppen substituierte Comonomere sind beispielsweise Acrylamid, Methacrylamid, Alkyl- und Dialkylacrylamid, Alkyl- und Dialkylmethacrylamid, Alkylacrylat, Alkylmethacrylat, Vinylcaprolacton, Vinylpyrrolidon, Vinylester, Vinylalkohol, Propylenglykol oder Ethylenglykol, aminsubstituierte Vinylmonomere wie z.B. Dialkylaminoalkylacrylat, Dialkylaminoalkylmethacrylat, Monoalkylaminoalkylacrylat und Monoalkylaminoalkylmethacrylat, wobei die Alkylgruppen dieser Monomere vorzugsweise C1- bis C7-Alkylgruppen, besonders bevorzugt C1- bis C3-Alkylgruppen sind.

Geeignete Polymere mit Säuregruppen sind insbesondere unvernetzte oder mit polyfunktionellen Agenzien vernetzte Homopolymere der Acrylsäure oder der Methacrylsäure, Copolymere der Acrylsäure oder Methacrylsäure mit Monomeren ausgewählt aus Acrylsäure- oder Methacrylsäureestern, Acrylamiden, Methacrylamiden und Vinylpyrrolidon, Homopolymere der Crotonsäure sowie Copolymere der Crotonsäure mit Monomeren ausgewählt aus Vinylestern, Acrylsäure- oder Methacrylsäureestern, Acrylamiden und Methacrylamiden. Ein geeignetes natürliches Polymer ist beispielsweise Schellack.

Bevorzugte Polymere mit Säuregruppen sind:
Terpolymere aus Acrylsäure, Alkylacrylat und N-Alkylacrylamid (INCI-Bezeichnung: Acrylates/Acrylamide Copolymer), insbesondere Terpolymere aus Acrylsäure, Ethylacrylat und N-tert-Butylacrylamid; vernetzte oder unvernetzte Vinylacetat/Crotonsäure Copolymere (INCI-Bezeichnung: VA/Crotonates Copolymer); Copolymere aus ein oder mehreren C1-C5-Alkylacrylaten, insbesondere C2-C4-Alkylacrylaten und mindestens einem Monomer ausgewählt aus Acrylsäure oder Methacrylsäure (INCI-Bezeichnung: Acrylates Copolymer), z.B. Terpolymere aus tert.-Butylacrylat, Ethylacrylat und Methacrylsäure; Natriumpolystyrolsulfonat; Vinylacetat/Crotonsäure/ Vinylalkanoat Copolymere, z.B. Copolymere aus Vinylacetat, Crotonsäure und Vinylpropionat; Copolymere aus Vinylacetat, Crotonsäure und Vinylneodecanoat (INCI-Bezeichnungen: VA/Crotonates/Vinyl Propionate Copolymer, VA/Crotonates/Vinyl Neodecanoate Copolymer); Aminomethylpropanol-Acrylat Copolymere; Copolymere aus Vinylpyrrolidon und mindestens einem weiteren Monomer ausgewählt aus Acrylsäure und Methacrylsäure sowei ggf. Acrylsäureestern und Methacrylsäureestern; Copolymere aus Methylvinylether und Maleinsäuremonoalkylestern (INCI-Bezeichnungen: Ethylester of PVM/MA Copolymer, Butylester of PVM/MA Copolymer); Aminomethylpropanolsalze von Copolymeren aus Allylmethacrylat und mindestens einem weiteren Monomer ausgewählt aus Acrylsäure, und Methacrylsäure sowei ggf. Acrylsäureestern und Methacrylsäureestern; vernetzte Copolymere aus Ethylacrylat und Methacrylsäure; Copolymere aus Vinylacetat, Mono-n-butylmaleat und Isobornylacrylat; Copolymere aus zwei oder mehr Monomeren ausgewählt aus Acrylsäure und Methacrylsäure sowie ggf. Acrylsäureestern und Methacrylsäureestern; Copolymere aus Octylacrylamid und mindestens einem Monomeren ausgewählt aus Acrylsäure und Methacrylsäure sowei ggf. Acrylsäureestern und Methacrylsäureestern; Polyester aus Diglycol, Cyclohexandimethanol, Isophtalsäure und Sulfoisophtalsäure, wobei die Alkylgruppen der vorstehend genannten Polymere in der Regel vorzugsweise 1, 2, 3 oder 4 C-Atome aufweisen.

In einer Ausführungsform enthält das erfindungsmäße Mittel als haarpflegenden oder haarfestigenden Wirkstoff mindestens ein zwitterionisches und/oder amphoteres Polymer in einer Menge von vorzugsweise 0,01 bis 20 Gew.% oder von 0,05 bis 10 Gew.%, besonders bevorzugt von 0,1 bis 5 Gew.%. Zwitterionische Polymere weisen gleichzeitig mindestens eine anionische und mindestens eine kationische Ladung auf. Amphotere Polymere weisen mindestens ein saure Gruppe (z.B. Carbonsäure- oder Sulphonsäuregruppe) und mindestens eine basische Gruppe (z.B. Aminogruppe) auf. Säuregruppen können mittels üblicher Basen wie z.B. organischer Amine oder Alkali- oder Erdalkalihydroxide deprotoniert sein.

Bevorzugte zwitterionische oder amphotere Polymere sind:
Copolymere gebildet aus Alkylacrylamid, Alkylaminoalkylmethacrylat und zwei oder mehr Monomeren aus Acrylsäure und Methacrylsäure sowie ggf. deren Estern, insbesondere Copolymere aus Octylacrylamid, Acrylsäure, Butylaminoethylmethacrylat, Methylmethacrylat und Hydroxypropylmethacrylat (INCI-Bezeichnung: Octylacrylamide/Acrylates/Butylaminoethyl Methacrylate Copolymer); Copolymere, welche gebildet sind aus mindestens einer ersten Monomerart, welche quaternäre Amingruppen aufweist und mindestens einer zweiten Monomerart, welche Säuregruppen aufweist; Copolymere aus Fettalkoholacrylaten, Alkylaminoxidmethacrylat und mindestens einem Monomeren ausgewählt aus Acrylsäure und Methacrylsäure sowie ggf. Acrylsäureestern und Methacrylsäureestern, insbesondere Copolymere aus Laurylacrylat, Stearylacrylat, Ethylaminoxidmethacrylat und mindestens einem Monomeren ausgewählt aus Acrylsäure und Methacrylsäure sowie ggf. deren Estern; Copolymere aus Methacryloylethylbetain und mindestens einem Monomeren ausgewählt aus Methacrylsäure und Methacrylsäureestern; Copolymere aus Acrylsäure, Methylacrylat und Methacrylamidopropyltrimethylammoniumchlorid (INCI-Bezeichnung: Polyquaternium-47); Copolymere aus Acrylamidopropyltrimethylammoniumchlorid und Acrylaten oder Copolymere aus Acrylamid, Acrylamidopropyltrimethylammoniumchlorid, 2-Amidopropylacrylamidsulfonat und Dimethylaminopropylamin (INCI-Bezeichnung: Polyquaternium-43); Oligomere oder Polymere, herstellbar aus quaternären Crotonbetainen oder quaternären Crotonbetainestern.

In einer Ausführungsform enthält das erfindungsmäße Mittel als haarpflegenden oder haarfestigenden Wirkstoff mindestens ein kationisches Polymer, d.h. ein Polymer mit kationischen oder kationisierbaren Gruppen, insbesondere primären, sekundären, tertiären oder quaternären Amingruppen in einer Menge von vorzugsweise 0,01 bis 20 Gew.% oder von 0,05 bis 10 Gew.%, besonders bevorzugt von 0,1 bis 5 Gew.%. Die kationische Ladungsdichte beträgt vorzugsweise 1 bis 7 meq/g.

Bei den geeigneten kationaktiven Polymeren handelt es sich vorzugsweise um haarfestigende oder um haarkonditionierende Polymere. Geeignete Polymere der Komponente (B) enthalten vorzugsweise quaternäre Amingruppen. Die kationischen Polymere können Homo- oder Copolymere sein, wobei die quaternären Stickstoffgruppen entweder in der Polymerkette oder vorzugsweise als Substituent an einem oder mehreren der Monomeren enthalten sind. Die Ammoniumgruppen enthaltenden Monomere können mit nicht kationischen Monomeren copolymerisiert sein. Geeignete kationische Monomere sind ungesättigte, radikalisch polymerisierbare Verbindungen, welche mindestens eine kationische Gruppe tragen, insbesondere ammoniumsubstituierte Vinylmonomere wie z.B. Trialkylmethacryloxyalkylammonium, Trialkylacryloxyalkylammonium, Dialkyldiallylammonium und quaternäre Vinylammoniummonomere nit cyclischen, kationische Stickstoffe enthaltenden Gruppen wie Pyridinium, Imidazolium oder quaternäre Pyrrolidone, z.B. Alkylvinylimidazolium, Alkylvinylpyridinium, oder Alkylvinylpyrrolidon Salze. Die Alkylgruppen dieser Monomere sind vorzugsweise niedere Alkylgruppen wie z.B. C1- bis C7-Alkylgruppen, besonders bevorzugt C1- bis C3-Alkylgruppen.

Die Ammoniumgruppen enthaltenden Monomere können mit nicht kationischen Monomeren copolymerisiert sein. Geeignete Comonomere sind beispielsweise Acrylamid, Methacrylamid, Alkyl- und Dialkylacrylamid, Alkyl- und Dialkylmethacrylamid, Alkylacrylat, Alkylmethacrylat, Vinylcaprolacton, Vinylcaprolactam, Vinylpyrrolidon, Vinylester, z.B. Vinylacetat, Vinylalkohol, Propylenglykol oder Ethylenglykol, wobei die Alkylgruppen dieser Monomere vorzugsweise C1- bis C7-Alkylgruppen, besonders bevorzugt C1- bis C3-Alkylgruppen sind.

Geeignete Polymere mit quaternären Amingruppen sind z.B. die im CTFA Cosmetic Ingredient Dictionary unter den Bezeichnungen Polyquaternium beschriebenen Polymere wie Methylvinylimidazoliumchlorid/Vinylpyrrolidon Copolymer (Polyquaternium-16) oder quaternisiertes Vinylpyrrolidon/Dimethylaminoethylmethacrylat Copolymer (Polyquaternium-11) sowie quaternäre Silikonpolymere bzw. -oligomere wie z.B. Silikonpolymere mit quaternären Endgruppen (Quaternium-80).

Bevorzugte kationische Polymere auf synthetischer Basis:
Poly(dimethyldiallylammoniumchlorid); Copolymere aus Acrylamid und Dimethyldiallylammoniumchlorid; quaternäre Ammoniumpolymere, gebildet durch die Reaktion von Diethylsulfat und einem Copolymer aus Vinylpyrrolidon und Dimethylaminoethylmethacrylat, insbesondere Vinylpyrrolidon/Dimethylaminoethylmethacrylatmethosulfat Copolymer (z.B. Gafquat® 755 N, Gafquat® 734); quaternäre Ammoniumpolymere aus Methylvinylimidazoliumchlorid und Vinylpyrrolidon (z.B. LUVIQUAT® HM 550); Polyquaternium-35; Polyquaternium-57; Polymer aus Trimethylammonium-ethyl-methacrylatchlorid; Terpolymere aus Dimethyldiallylammoniumchlorid, Natriumacrylat und Acrylamid (z.B. Merquat® Plus 3300); Copolymere aus Vinylpyrrolidon, Dimethylaminopropylmethacrylamid und Methacryloylaminopropyllauryldimethylammoniumchlorid; Terpolymere aus Vinylpyrrolidon, Dimethylaminoethylmethacrylat und Vinylcaprolactam (z.B. Gaffix® VC 713); Vinylpyrrolidon/Methacrylamidopropyltrimethylammoniumchlorid Copolymere (z.B. Gafquat® HS 100); Copolymere aus Vinylpyrrolidon und Dimethylaminoethylmethacrylat; Copolymere aus Vinylpyrrolidon, Vinylcaprolactam und Dimethylaminopropylacrylamid; Poly- oder Oligoester, aufgebaut aus mindestens einer ersten Monomerart, die ausgewählt ist aus mit mindestens einer quaternären Ammoniumgruppe substituierten Hydroxysäure; endständig mit quaternären Ammoniumgruppen substituierte Dimethylpolysiloxane.

Geeignete kationische Polymere, die von natürlichen Polymeren abgeleitet sind, sind insbesondere kationische Derivate von Polysacchariden, beispielsweise kationische Derivate von Cellulose, Stärke oder Guar. Geeignet sind weiterhin Chitosan und Chitosanderivate. Kationische Polysaccharide haben z.B. die allgemeine Formel

G-O-B-N⁺R^{a}R^{b}R^{c} X⁻

G ist ein Anhydroglucoserest, beispielsweise Stärke- oder Celluloseanhydroglucose;
B ist eine divalente Verbindungsgruppe, beispielsweise Alkylen, Oxyalkylen, Polyoxyalkylen oder Hydroxyalkylen;
R^{a}, R^{b} und R^{c} sind unabhängig voneinander Alkyl, Aryl, Alkylaryl, Arylalkyl, Alkoxyalkyl oder Alkoxyaryl mit jeweils bis zu 18 C-Atomen, wobei die Gesamtzahl der C-Atome in R^{a}, R^{b} und R^{c} vorzugsweise maximal 20 ist;
X ist ein übliches Gegenanion, beispielsweise ein Halogen, Acetat, Phosphat, Nitrat oder Alkylsulfat, vorzugsweise ein Chlorid. Kationische Cellulosen sind z.B. solche mit den INCI-Bezeichnungen Polyquaternium-10 oder Polyquaternium-24. Ein geeignetes kationisches Guarderivat hat z.B. die INCI-Bezeichnung Guar Hydroxypropyltrimonium Chloride.

Besonders bevorzugte kationaktive Stoffe sind Chitosan, Chitosansalze und Chitosan-Derivate. Bei den erfindungsgemäß einzusetzenden Chitosanen handelt es sich um vollständig oder partiell deacetylierte chitine. Das Molekulargewicht kann über ein breites Spektrum verteilt sein, beispielsweise von 20.000 bis ca. 5 Millionen g/mol, z.B. von 30.000 bis 70.000 g/mol. Vorzugsweise liegt das Molekulargewicht jedoch über 100.000 g/mol, besonders bevorzugt von 200.000 bis 700.000 g/mol. Der Deacetylierungsgrad beträgt vorzugsweise 10 bis 99%, besonders bevorzugt 60 bis 99%. Ein bevorzugtes Chitosansalz ist Chitosoniumpyrrolidoncarboxylat, z.B. Kytamer® PC mit einem Molekulargewicht von ca. 200.000 bis 300.000 g/mol und Deacetylierung von 70 bis 85%. Als Chitosanderivate kommen quaternierte, alkylierte oder hydroxyalkylierte Derivate, z.B. Hydroxyethyl-, Hydroxypropyl- oder Hydroxybutylchitosan in Betracht. Die Chitosane oder Chitosanderivate liegen vorzugsweise in neutralisierter oder partiell neutralisierter Form vor. Der Neutralisationsgrad liegt vorzugsweise bei mindestens 50%, besonders bevorzugt zwischen 70 und 100%, bezogen auf die Anzahl der freien Basengruppen. Als Neutralisationsmittel können prinzipiell alle kosmetisch verträglichen anorganischen oder organischen Säuren verwendet werden wie beispielsweise Ameisensäure, Weinsäure, Äpfelsäure, Milchsäure, Zitronensäure, Pyrrolidoncarbonsäure, Salzsäure u.a., von denen die Salzsäure und Ameisensäure besonders bevorzugt sind.

Bevorzugte kationische Polymere auf natürlicher Basis: kationische Cellulosederivate aus Hydroxyethylcellulose und Diallyldimethylammoniumchlorid; kationische Cellulosederivate aus Hydroxyethylcellulose und mit Trimethylammonium substituiertem Epoxid; Chitosan und dessen Salze; Hydroxyalkylchitosane und deren Salze; Alkyl-hydroxyalkylchitosane und deren Salze; N-Hydroxyalkylchitosanalkylether.

In einer weiteren bevorzugten Ausführungsform enthält das erfindungsgemäße Mittel 0,01 bis 15 Gew.%, vorzugsweise 0,5 bis 10 Gew.% mindestens eines synthetischen oder natürlichen nichtionischen filmbildenden Polymers. Unter natürlichen Polymeren werden auch chemisch modifizierte Polymere natürlichen Ursprungs verstanden. Unter filmbildenden Polymeren werden solche Polymere verstanden, die bei Anwendung in 0,01 bis 5%iger wässriger, alkoholischer oder wässrig-alkoholischer Lösung in der Lage sind, auf dem Haar einen Polymerfilm abzuscheiden.

Geeignete synthetische, nichtionische filmbildende, haarfestigende Polymere sind Homo- oder Copolymere, die aus mindestens einem der folgenden Monomere aufgebaut sind: Vinylpyrrolidon, Vinylcaprolactam, Vinylester wie z.B. Vinylacetat, Vinylalkohol, Acrylamid, Methacrylamid, Alkyl- und Dialkylacrylamid, Alkyl- und Dialkylmethacrylamid, Alkylacrylat, Alkylmethacrylat, Propylenglykol oder Ethylenglykol, wobei die Alkylgruppen dieser Monomere vorzugsweise C1- bis C7-Alkylgruppen, besonders bevorzugt C1- bis C3-Alkylgruppen sind. Geeignet sind z.B. Homopolymere des Vinylcaprolactams, des Vinylpyrrolidons oder des N-Vinylformamids. Weitere geeignete synthetische filmbildende, nicht-ionische, haarfestigende Polymere sind z.B. Copolymerisate aus Vinylpyrrolidon und Vinylacetat, Terpolymere aus Vinylpyrrolidon, Vinylacetat und Vinylpropionat, Polyacrylamide; Polyvinylalkohole sowie Polyethylenglykol/Polypropylenglykol Copolymere. Geeignete natürliche filmbildende Polymere sind z.B. Cellulosederivate, z.B. Hydroxyalkylcellulose.

Bevorzugte nichtionische Polymnere sind:
Polyvinylpyrrolidon, Polyvinylcaprolactam, Vinylpyrrolidon/Vinylacetat Copolymere, Polyvinylalkohol, Isobutylen/Ethylmaleimid/Hydroxyethylmaleimid Copolymer; Copolymyere aus Vinylpyrrolidon, Vinylacetat und Vinylpropionat.

In einer Ausführungsform enthält das erfindungsmäße Mittel als haarpflegenden Wirkstoff mindestens eine Silikonverbindung in einer Menge von vorzugsweise 0,01 bis 15 Gew.%, besonders bevorzugt von 0,1 bis 5 Gew.%. Die Silikonverbindungen umfassen flüchtige und nicht-flüchtige Silikone und in dem Mittel lösliche und unlösliche Silikone. Bei einer Ausführungsform handelt es sich um hochmolekulare Silikone mit einer Viskosität von 1.000 bis 2.000.000 cSt bei 25°C, vorzugsweise 10.000 bis 1.800.000 oder 100.000 bis 1.500.000. Die Silikonverbindungen umfassen Polyalkyl- und Polyarylsiloxane, insbesondere mit Methyl-, Ethyl-, Propyl-, Phenyl-, Methylphenyl- und Phenylmethylgruppen. Bevorzugt sind Polydimethylsiloxane, Polydiethylsiloxane, Polymethylphenylsiloxane. Bevorzugt sind auch glanzgebende, arylierte Silikone mit einem Brechungsindex von mindestens 1,46, oder mindestens 1,52. Die Silikonverbindungen umfassen insbesondere die Stoffe mit den INCI-Bezeichnungen Cyclomethicone, Dimethicone, Dimethiconol, Dimethicone Copolyol, Phenyl Trimethicone, Amodimethicone, Trimethylsilylamodimethicone, Stearyl Siloxysilicate, Polymethylsilsesquioxane, Dimethicone Crosspolymer. Geeignet sind auch Silikonharze und Silikonelastomere, wobei es sich um hochvernetzte Siloxane handelt.
Bevorzugte Silikone sind:
cyclische Dimethylsiloxane, lineare Polydimethylsiloxane, Blockpolymere aus Polydimethylsiloxan und Polyethylenoxid und/oder Polypropylenoxid, Polydimethylsiloxane mit end- oder seitenständigen Polyethylenoxid- oder Polypropylenoxidresten, Polydimethylsiloxane mit endständigen Hydroxylgruppen, phenylsubstituierte Polydimethylsiloxane, Silikonemulsionen, Silkonelastomere, Silikonwachse, Silikongums und aminosubstituierte Silikone.

In einer Ausführungsform enthält das erfindungsmäße Mittel als haarschützenden Wirkstoff einen Lichtschutzstoff in einer Menge von vorzugsweise 0,01 bis 10 Gew.% oder von 0,1 bis 5 Gew.%, besonders bevorzugt von 0,2 bis 2 Gew.%. Die Lichtschutzstoffe umfassen insbesondere alle in der EP 1 084 696 genannten Lichtschutzstoffe. Bevorzugt sind: 4-Methoxy-zimtsäure-2-ethylhexylester, Methylmethoxycinnamat, 2-Hydroxy-4-methoxy-benzophenon-5-sulfonsäure und polyethoxylierte p-Aminobenzoate.

In einer Ausführungsform enthält das erfindungsmäße Mittel als haarpflegenden bzw. haarfestigenden Wirkstoff mindestens ein hydrophobes Öl oder Wachs in einer Menge von vorzugsweise 0,01 bis 20 oder von 0,05 bis 10, besonders bevorzugt von 0,1 bis 5 Gew.%.
Die flüssigen, hydrophoben Öle haben einen Schmelzpunkt von kleiner oder gleich 25°C und einen Siedepunkt von vorzugsweise über 250°C, insbesondere über 300 °C. Hierfür kann prinzipiell jedes dem Fachmann allgemein bekannte Öl eingesetzt werden. In Frage kommen pflanzliche oder tierische Öle, Mineralöle (Paraffinum liquidum), Silikonöle oder deren Mischungen. Geeignet sind Kohlenwasserstofföle, z.B. Paraffin- oder Isoparaffinöle, Squalan, Öle aus Fettsäuren und Polyolen, insbesondere Triglyceride. Geeignete pflanzliche Öle sind z.B. Sonnenblumenöl, Kokosöl, Rizinusöl, Lanolinöl, Jojobaöl, Maisöl, Sojaöl.

Als Wachs oder wachsartiger Stoff kann prinzipiell jedes im Stand der Technik bekannte Wachs eingesetzt werden. Hierzu zählen tierische, pflanzliche, mineralische und synthetische Wachse, mikrokristalline Wachse, makrokristalline Wachse, feste Paraffine, Petrolatum, Vaseline, Ozokerit, Montanwachs, Fischer-Tropsch-Wachse, Polyolefinwachse z.B. Polybuten, Bienenwachs, Wollwachs und dessen Derivate wie z.B. Wollwachsalkohole, Candelillawachs, Olivenwachs, Carnaubawachs, Japanwachs, Apfelwachs, gehärtete Fette, Fettsäureester und Fettsäureglyceride mit einem Erstarrungspunkt von jeweils oberhalb 40°C, Polyethylenwachse und Silikonwachse. Die Wachse oder wachsartigen Stoffe haben einen Erstarrungspunkt oberhalb 40°C, vorzugsweise oberhalb 55°C. Die Nadelpenetrationszahl (0,1 mm, 100 g, 5 s, 25°C; nach DIN 51 579) liegt vorzugsweise im Bereich von 2 bis 70, insbesondere von 3 bis 40.

In einer Ausführungsform enthält das erfindungsmäße Mittel 0,01 bis 20, besonders bevorzugt von 0,05 bis 10, ganz besonders bevorzugt von 0,1 bis 5 Gew.% an mindestens einem haarkonditionierenden Zusatzstoff, ausgewählt aus Betain; Panthenol; Panthenylethylether; Sorbitol; Proteinhydrolysaten; Pflanzenextrakten; A-B-Block-Copolymeren aus Alkylacrylaten und Alkylmethacryaten; A-B-Block-Copolymeren aus Alkylmethacrylaten und Acrylnitril; A-B-A-Block-Copolymeren aus Lactid und Ethylenoxid; A-B-A-Block-Copolymeren aus Caprolacton und Ethylenoxid; A-B-C-Block-Copolymeren aus Alkylen- oder Alkadienverbindungen, Styrol und Alkylmethacrylaten; A-B-C-Block-Copolymeren aus Acrylsäure, Styrol und Alkylmethacrylaten; sternförmigen Block-Copolymeren; hyperverzweigten Polymeren; Dendrimeren; intrinsisch elektrisch leitfähigen 3,4-Polyethylendioxythiophenen und intrinsisch elektrisch leitfähigen Polyanilinen.

In einer Ausführungsform enthält das erfindungsmäße Mittel als haarpflegenden und/oder haarreinigenden Wirkstoff 0,01 bis 20, besonders bevorzugt von 0,05 bis 10, ganz besonders bevorzugt von 0,1 bis 5 Gew.% an mindestens einem haarpflegenden und/oder haarreinigenden Tensid. Das Tensid kann nichtionisch, anionisch, kationisch oder zwitterionisch sein.

Geeignete nichtionische Tenside sind z.B.
- Ethoxylierte Fettalkohole, Fettsäuren, Fettsäureglyceride oder Alkylphenole, insbesondere Anlagerungsprodukte von 2 bis 30 mol Ethylenoxid und/oder 1 bis 5 mol Propylenoxid an C8- bis C22-Fettalkohole, an C12- bis C22-Fettsäuren oder an Alkylphenole mit 8 bis 15 C-Atomen in der Alkylgruppe
- C12- bis C22-Fettsäuremono- und -diester von Anlagerungsprodukten von 1 bis 30 mol Ethylenoxid an Glycerin
- Anlagerungsprodukte von 5 bis 60 mol Ethylenoxid an Rizinusöl oder an gehärtetes (hydriertes) Rizinusöl.
- Fettsäurezuckerester, insbesondere Ester aus Saccharose und ein oder zwei C8- bis C22-Fettsäuren, INCI: Sucrose Cocoate, Sucrose Dilaurate, Sucrose Distearate, Sucrose Laurate, Sucrose Myristate, Sucrose Oleate, Sucrose Palmitate, Sucrose Ricinoleate, Sucrose Stearate
- Ester aus Sorbitan und ein, zwei oder drei C8- bis C22-Fettsäuren und einem Ethoxylierungsgrad von 4 bis 20
- Polyglycerylfettsäureester, insbesondere aus ein, zwei oder mehreren C8- bis C22-Fettsäuren und Polyglycerin mit vorzugsweise 2 bis 20 Glyceryleinheiten
- Alkylglukoside, Alkyloligoglukoside und Alkylpolyglucoside mit C8- bis C22-Alkylgruppen, z.B. Decyl Glucoside oder Lauryl Glucoside,

Geeignete anionische Tenside sind z.B. Salze und Ester von Carbonsäuren, Alkylethersulfate und Alkylsulfate, Fettalkoholethersulfate, Sulfonsäure und ihre Salze (z.B. Sulfosuccinate oder Fettsäureisethienate), Phosphorsäureester und ihre Salze, Acylaminosäuren und ihre Salze. Eine ausführliche Beschreibung dieser anionischen Tenside ist der Publikation "FIEDLER - Lexikon der Hilfsstoffe", Band 1, fünfte Auflage (2002), Seiten 97 bis 102, zu entnehmen, auf die hiermit ausdrücklich Bezug genommen wird. Bevorzugte Tenside sind Mono-, Di- und/oder Triester der Phosphorsäure mit Anlagerungsprodukten von 2 bis 30 mol Ethylenoxid an C8- bis C22-Fettalkohole.

Geeignete amphotere Tenside sind z.B. Derivate aliphatischer quaternärer Ammonium-, Phosphonium- und Sulfoniumverbindungen der Formel 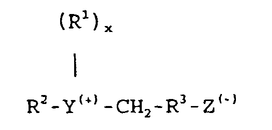 wobei R1 eine geradkettige oder verzweigtkettige Alkyl-, Alkenyl- oder Hydroxyalkylgruppe mit 8 bis 18 C-Atomen und 0 bis etwa 10 Ethylenoxideinheiten und 0 bis 1 Glycerineinheit darstellt; Y eine N-, P- oder S-haltige Gruppe ist; R2 eine Alkyl- oder Monohydroxyalkylgruppe mit 1 bis 3 C-Atomen ist; X gleich 1 ist, falls Y ein Schwefelatom ist und X gleich 2 ist, wenn Y ein Stickstoffatom oder ein Phosphoratom ist; R3 eine Alkylen- oder Hydroxyalkylengruppe mit 1 bis 4 C-Atomen ist und Z⁽⁻⁾ eine Carboxylat-, Sulfat-, Phosphonat- oder Phosphatgruppe darstellt.
Andere amphotere Tenside wie Betaine sind ebenso geeignet. Beispiele für Betaine umfassen C8- bis C18-Alkylbetaine wie Cocodimethylcarboxymethylbetain, Lauryldimethylcarboxymethylbetain, Lauryldimethylalphacarboxyethylbetain, Cetyldimethylcarboxymethylbetain, Oleyldimethylgammacarboxypropylbetain und Lauryl-bis(2-hydroxypropyl)alphacarboxyethylbetain; C8- bis C18-Sulfobetaine wie Cocodimethylsulfopropylbetain, Stearyldimethylsulfopropylbetain, Lauryldimethylsulfoethylbetain, Laurylbis-(2-hydroxyethyl)sulfopropylbetain; die Carboxylderivate des Imidazols, die C8- bis C18-Alkyldimethylammoniumacetate, die C8- bis C18-Alkyldimethylcarbonylmethylammoniumsalze sowie die C8- bis C18-Fettsäurealkylamidobetaine wie beispielsweise Kokosfettsäureamidopropylbetain und N-Kokosfettsäureamidoethyl-N-[2-(carboxymethoxy)-ethyl]-glycerin (CTFA-Name: Cocoamphocarboxyglycinate).

Geeignete kationische Tenside enthalten Aminogruppen oder quaternisierte hydrophile Ammoniumgruppen, welche in Lösung eine positive Ladung tragen und durch die allgemeine Formel

N⁽⁺⁾R¹R²R³R⁴ X⁽⁻⁾

dargestellt werden können, wobei R1 bis R4 unabhängig voneinander aliphatische Gruppen, aromatische Gruppen, Alkoxygruppen, Polyoxyalkylengruppen, Alkylamidogruppen, Hydroxyalkylgruppen, Arylgruppen oder Alkarylgruppen mit 1 bis 22 C-Atomen bedeuten, wobei mindestens ein Rest mindestens 6, vorzugsweise mindestens 8 C-Atome aufweist und X- ein Anion darstellt, beispielsweise ein Halogen, Acetat, Phosphat, Nitrat oder Alkylsulfat, vorzugsweise ein Chlorid. Die aliphatischen Gruppen können zusätzlich zu den C-Atomen und den Wasserstoffatomen auch Querverbindungen oder andere Gruppen wie beispielsweise weitere Aminogruppen enthalten. Beispiele für geeignete kationische Tenside sind die Chloride oder Bromide von Alkyldimethylbenzylammoniumsalzen, Alkyltrimethylammoniumsalze, beispielsweise Cetyltrimethylammoniumchlorid oder -bromid, Tetradecyltrimethylammoniumchlorid oder -bromid, Alkyldimethylhydroxyethylammoniumchloride oder -bromide, die Dialkyldimethylammoniumchloride oder -bromide, Alkylpyridiniumsalze, beispielsweise Lauryl- oder Cetylpyridiniumchlorid, Alkylamidoethyltrimethylammoniumethersulfate sowie Verbindungen mit kationischem Charakter wie Aminoxide, beispielsweise Alkylmethylaminoxide oder Alkylaminoethyldimethylaminoxide. Besonders bevorzugt sind C8-22-Alkyldimethylbenzylammoniumverbindungen, C8-22- Alkyltrimethylammoniumverbindungen, insbesondere Cetyltrimethylammoniumchlorid, C8-22-Alkyldimethylhydroxyethylammoniumverbindungen, Di-(C8-22-alkyl)-dimethylammoniumverbindungen, C8-22-Alkylpyridiniumsalze, C8-22- Alkylamidoethyltrimethylammoniumethersulfate, C8-22- Alkylmethylaminoxide, C8-22-Alkylaminoethyldimethylaminoxide.

Eine besondere Ausführungsform der Erfindung betrifft ein Haarpflegemittel. Haarpflegemittel sind z.B. Conditioner, Treatmants, Haarkuren, Spülungen oder dergleichen. Das Haarpflegemittel enthält mindestens einen haarpflegenden Wirkstoff ausgewählt aus den oben genannten Silikonverbindungen, kationischen oder aminsubstituierten Tensiden und kationischen oder aminsubstituierten Polymeren. Der haarpflegende Wirkstoff kann in Mengen zwischen 0,01 und 10,0 Gew.%, insbesondere zwischen 0,01 und 5,0 Gew.%, bezogen auf das fertige Produkt, eingesetzt werden. Das erfindungsgemäße Haarpflegemittel kann nach Applikation auf das trockene, feuchte oder nasse Haar entweder im Haar verbleiben oder es kann nach einer geeigneten Einwirkzeit wieder ausgespült werden. Die Einwirkzeiten hängen von der Art des Haares ab. Als allgemeine Richtlinie kann von Einwirkzeiten zwischen 0,5 und 30 Minuten, insbesondere 0,5 und 10 Minuten, vorzugsweise zwischen 1 und 5 Minuten ausgegangen werden.

Neben den oben genannten kationischen Tensiden sind weitere geeignete kationische oder aminsubstituierte Tenside solche der Formel

R1-NH-(CH₂)n-NR2R3

oder der Formel

R1-NH- (CH₂)n-N⁺R2R3R4 X⁻

worin R1 ein Acyl- oder ein Alkylrest mit 8 bis 24 C-Atomen ist, welcher verzweigt oder unverzweigt, gesättigt oder ungesättigt sein kann, wobei der Acyl- und/oder der Alkylrest eine oder mehrere OH-Gruppen enthalten kann, R2, R3 und R4 unabhängig voneinander Wasserstoff, Alkyl- oder Alkoxyalkylreste mit 1 bis 6 C-Atomen sind, welche gleich oder verschieden, gesättigt oder ungesättigt und mit einer oder mehreren Hydroxygruppen substituiert sein können, X⁻ ein Anion ist, insbesondere ein Halogenidion oder eine Verbindung der allgemeinen Formel RSO₃⁻ , worin R die Bedeutung von gesättigten oder ungesättigten Alkylresten mit 1 bis 4 C-Atomen hat, und n eine ganze Zahl zwischen 1 und 10, vorzugsweise von 2 bis 5 bedeutet.

Vorzugsweise ist der haarpflegende Wirkstoff ein Amidoamin und/oder ein quaternisiertes Amidoamin der oben genannten Formeln, worin R1 ein verzweigter oder unverzweigter, gesättigter oder ungesättigter Acylrest mit 8 bis 24 C-Atomen ist, welcher mindestens eine OH-Gruppe enthalten kann. Bevorzugt sind auch solche Amine und/oder quaternisierte Amine, in denen mindestens einer der Reste R2, R3 und R4 ein Rest gemäß der allgemeinen Formel CH₂CH₂OR5 bedeuten, worin R5 die Bedeutung von Alkylresten mit 1 bis 4 C-Atomen, Hydroxyethyl oder H haben kann. Geeignete Amine oder Amidoamine, welche gegebenenfalls quaternisiert sein können, sind insbesondere solche mit den INCI-Bezeichnungen Ricinoleamidopropyl Betaine, Ricinoleamidopropyl Dimethylamine, Ricinoleamidopropyl Dimethyl Lactate, Ricinoleamidopropyl Ethyldimonium Ethosulfate, Ricinoleamidopropyltrimonium Chloride, Ricinoleamidopropyltrimonium methosulfate, Cocamidopropyl Betaine, Cocamidopropyl Dimethylamine, Cocamidopropyl Ethyldimonium Ethosulfate, Cocamidopropyltrimonium Chloride, Behenamidopropyl Dimethylamine, Isostearylamidopropyl Dimethylamine, Stearylamidopropyl Dimethylamine, Quaternium-33, Undecyleneamidopropyltrimonium Methosulfate.

In einer weiteren Ausführungsform enthält das erfindungsgemäße Mittel mindestens ein haarfärbendes Pigment, welches dem Haar Farb- und/oder Glanzeffekte verleiht. Die Farb- oder Glanzeffekte am Haar sind vorzugsweise temporär, d.h. sie halten bis zur nächsten Haarwäsche und können durch Waschen der Haare mit üblichen Shampoos wieder entfernt werden. Die Pigmente liegen in der Produktmasse in ungelöster Form vor und können in einer Menge von 0,01 bis 25 Gew.%, besonders bevorzugt von 5 bis 15 Gew.% enthalten sein. Die bevorzugte Teilchengröße beträgt 1 bis 200 *µ*m, insbesondere 3 bis 150 *µ*m, besonders bevorzugt 10 bis 100 *µ*m. Die Pigmente sind im Anwendungsmedium praktisch unlösliche Farbmittel und können anorganisch oder organisch sein. Auch anorganisch-organische Mischpigmente sind möglich. Bevorzugt sind anorganische Pigmente. Der Vorteil der anorganischen Pigmente ist deren ausgezeichnete Licht-, Wetter- und Temperaturbeständigkeit. Die anorganischen Pigmente können natürlichen Ursprungs sein, beispielsweise hergestellt aus Kreide, Ocker, Umbra, Grünerde, gebranntem Terra di Siena oder Graphit. Bei den Pigmenten kann es sich um Weißpigmente wie z.B. Titandioxid oder Zinkoxid, um Schwarzpigmente wie z.B. Eisenoxidschwarz, Buntpigmente wie z.B. Ultramarin oder Eisenoxidrot, um Glanzpigmente, Metalleffekt-Pigmente, Perlglanzpigmente sowie um Fluoreszenz- oder Phosphoreszenzpigmente handeln, wobei vorzugsweise mindestens ein Pigment ein farbiges, nicht-weißes Pigment ist. Geeignet sind Metalloxide, -hydroxide und -oxidhydrate, Mischphasenpigmente, schwefelhaltige Silicate, Metallsulfide, komplexe Metallcyanide, Metallsulfate, Metallchromate und -molybdate sowie die Metalle selbst (Bronzepigmente). Geeignet sind insbesondere Titandioxid (CI 77891), schwarzes Eisenoxid (CI 77499), gelbes Eisenoxid (CI 77492), rotes und braunes Eisenoxid (CI 77491), Manganviolett (CI 77742), Ultramarine (Natrium-Aluminiumsulfosilikate, CI 77007, Pigment Blue 29), Chromoxidhydrat (CI77289), Eisenblau (Ferric Ferrocyanide, CI77510), Carmine (Cochineal).

Besonders bevorzugt sind Perlglanz- und Farbpigmente auf Mica- bzw. Glimmerbasis welche mit einem Metalloxid oder einem Metalloxychlorid wie Titandioxid oder Wismutoxychlorid sowie gegebenenfalls weiteren farbgebenden Stoffen wie Eisenoxiden, Eisenblau, Ultramarine, Carmine etc. beschichtet sind und wobei die Farbe durch Variation der Schichtdicke bestimmt sein kann. Derartige Pigmente werden beispielsweise unter den Handelsbezeichnungen Ron®, Colorona®, Dichrona® und Timiron® von der Firma Merck, Deutschland vertrieben.

Organische Pigmente sind beispielsweise die natürlichen Pigmente Sepia, Gummigutt, Knochenkohle, Kasseler Braun, Indigo, Chlorophyll und andere Pflanzenpigmente. Synthetische organische Pigmente sind beispielsweise Azo-Pigmente, Anthrachinoide, Indigoide, Dioxazin-, Chinacridon-, Phtalocyanin-, Isoindolinon-, Perylen- und Perinon-, Metallkomplex-, Alkaliblau- und Diketopyrrolopyrrol-Pigmente.

Eine weitere Ausführungsform betrifft ein Mittel zur dauerhaften Verformung des Haares. Es enthält mindestens ein Reduktionsmittel, insbesondere eine keratinreduzierenden Mercaptoverbindung in einer Menge von vorzugsweise 0,5 bis 15 Gew.%. Das Dauerwellmittel liegt bevorzugt als wässrige, alkalische (pH = 5 bis 10) eingestellte Zubereitung vor, welche als keratinreduzierende Mercaptoverbindung, z.B. Cystein, Cysteamin, N-Acetyl-L-Cystein, Mercaptocarbonsäuren, wie z.B. Thioglykolsäure oder Thiomilchsäure, oder Salze von Mercaptocarbonsäuren, wie z.B. Ammonium- und Guanidinsalze der Thioglykolsäure oder Thiomilchsäure enthält. Die erforderliche Alkalität wird hierbei durch die Zugabe von Ammoniak, organischen Aminen, Ammonium- und Alkalicarbonaten oder -hydrogencarbonaten eingestellt. Es kommt aber auch ein neutral oder sauer (pH = 4,5 bis 7) eingestelltes Haarverformungsmittel in Betracht, das im wässrigen Medium einen wirksamen Gehalt an Sulfiten oder Mercaptocarbonsäureestern aufweist. Im ersteren Fall werden vorzugsweise Natrium- oder Ammoniumsulfit oder das Salz der schwefligen Säure mit einem organischen Amin, wie z.B. Monoethanolamin und Guanidin, in einer Konzentration von etwa 2 bis 12 Gew.% (berechnet als SO2) verwendet. Im letzteren Fall kommen insbesondere Thioglykolsäuremonoglykolester oder -glycerinester in einer Konzentration von etwa 5 bis 50 Gew.% (entsprechend einem Gehalt an freier Thioglykolsäure von 2 bis 16 Gew.%) zur Anwendung. Das erfindungsgemässe Mittel zur dauerhaften Haarverformung kann auch ein Gemisch der vorstehend genannten keratinreduzierenden Verbindungen enthalten. Nach einer für die dauerhafte Verformung des Haares ausreichenden Einwirkungszeit wird das Haar mit Wasser gespült und anschliessend öxidativ nachbehandelt (fixiert). Für die oxidative Nachbehandlung kann ein erfindungsgemässes, mindestens ein Oxidationsmittel enthaltendes Fixiermittel oder jedes beliebige bisher für eine derartige Behandlung verwendete Fixiermittel verwendet werden. Beispiele für in einem derartigen Fixiermittel verwendbare Oxidationsmittel sind Natrium- und Kaliumbromat, Natriumperborat, Harnstoffperoxid und Hydrogenperoxid. Die Konzentration des Oxidationsmittels kann dabei von etwa 0,5 bis 10 Gew.% betragen, wobei ein erfindungsgemässes Fixiermittel vorzugsweise 0,05 bis 5 Gew.% der sphärischen Partikel enthält. Sowohl das erfindungsgemässe Mittel zur dauerhaften Haarverformung als auch das erfindungsgemässe Fixiermittel kann in Form einer wässrigen Lösung oder Emulsion sowie in verdickter Form auf wässriger Basis, insbesondere als Creme, Gel oder Paste vorliegen. Es ist ebenfalls möglich, dieses Mittel unter Druck in Aerosoldosen abzufüllen und daraus als Schaum zu entnehmen. Das vorstehend beschriebene Verfahren zur dauerhaften Haarverformung ermöglicht eine schonende und gleichmässige Umformung vom Haaransatz bis zu den Haarspitzen, das Haar zeigt eine hervorragende Nass- und Trockenkämmbarkeit, einen angenehmen Griff und einen ansprechenden Glanz im getrockneten Zustand sowie eine lockere, sprunghafte und gleichmässige Dauerwellung, insbesondere im Bereich der Haarspitzen.

Eine weitere Ausführungsform betrifft ein Haarfärbemittel. Haartönungsmittel. Es kann sowohl in Form eines oxidativen als auch nicht-oxidativen Färbemittels auf Basis der nachstehend genannten oxidativen und/oder direktziehenden Farbstoffe vorliegen. Die Gesamtmenge der in dem erfindungsgemäßen Mittel enthaltenen Oxidationsfarbstoffvorstufen beträgt vorzugsweise etwa 0,01 bis 12 Gew.%, insbesondere etwa 0,2 bis 6 Gew.%. Als geeignete Oxidationsfarbstoffvorstufen können beispielsweise die folgenden Entwicklersubstanzen und Kupplersubstanzen und mit sich selbst kuppelnden Verbindungen genannt werden.

### Entwickler:

1,4-Diamino-benzol (p-Phenylendiamin), 1,4-Diamino-2-methylbenzol (p-Toluylendiamin), 1,4-Diamino-2-(thiophen-2-yl)-benzol, 1,4-Diamino-2-(thiophen-3-yl)benzol, 1,4-Diamino-2-(pyridin-3-yl)benzol, 2,5-Diaminobiphenyl, 1,4-Diamino-2-methoxymethyl-benzol, 1,4-Diamino-2-aminomethyl-benzol, 1,4-Diamino-2-hydroxymethyl-benzol, 4-[Di(2-hydroxyethyl)amino]-anilin, 1,4-Diamino-2-(1-hydroxyethyl)-benzol, 1,4-Diamino-2-(2-hydroxyethyl)-benzol, 1,3-Bis[(4-aminophenyl)(2-hydroxyethyl)amino]-2-propanol, 1,8-Bis(2,5-diaminophenoxy)-3,6-dioxaoctan, 2,5-Diamino-4'-hydroxy-1,1'-biphenyl, 2,5-Diamino-2'-trifluormethyl-1,1'-biphenyl, 2,4',5-Triamino-1,1'-biphenyl, 4-Amino-phenol, 4-Amino-3-methyl-phenol, 4-Methylamino-phenol, 4-Amino-2-(aminomethyl)-phenol, 4-Amino-2-[(2-hydroxyethyl)-amino]methyl-phenol, 4-Amino-2-(methoxymethyl)-phenol, 5-Amino-salicylsäure, 2,4,5,6-Tetraaminopyrimidin, 2,5,6-Triamino-4-(1H)-pyrimidon, 4,5-Diamino-1-(2-hydroxyethyl)-1H-pyrazol, 4,5-Diamino-1-pentyl-1H-pyrazol, 4,5-Diamino-1-(phenylmethyl)-1H-pyrazol, 4,5-Diamino-1-((4-methoxyphenyl)methyl-1H-pyrazol, 2-Amino-phenol, 2-Amino-6-methyl-phenol, 2-Amino-5-methyl-phenol, 1,2,4-Trihydroxy-benzol, 2,4-Diaminophenol, 1,4-Dihydroxybenzol, 2-(((4-Aminophenyl)amino)methyl)-1,4-diaminobenzol.

### Kuppler:

N-(3-Dimethylamino-phenyl)-harnstoff, 2,6-Diamino-pyridin, 2-Amino-4-[(2-hydroxyethyl)amino]-anisol, 2,4-Diamino-1-fluor-5-methyl-benzol, 2,4-Diamino-1-ethoxy-5-methyl-benzol, 2,4-Diamino-1-(2-hydroxyethoxy)-5-methyl-benzol, 3-Amino-6-methoxy-2-(methylamino)-pyridin, 3,5-Diamino-2,6-dimethoxypyridin, 1,3-Diamino-benzol, 2,4-Diamino-1-(2-hydroxyethoxy)-benzol, 1,3-Diamino-4-(3-hydroxypropoxy)-benzol, 1,3-Diamino-4-(2-methoxyethoxy)-benzol, 1,3-Di(2,4-diaminophenoxy)-propan, 2,6-Bis(2-hydroxyethyl)amino-toluol, 5-Amino-2-methyl-phenol, 5-Amino-4-fluor-2-methyl-phenol, 3-Amino-2,4-dichlor-phenol, 3-Amino-2-chlor-6-methyl-phenol, 3-Amino-phenol, 5-[(2-Hydroxyethyl)amino]-2-methyl-phenol, 2-Amino-3-hydroxy-pyridin, 2,6-Dihydroxy-3,4-dimethylpyridin, 5-Amino-4-chlor-2-methyl-phenol, 1-Naphthol, 1,5-Dihydroxy-naphthalin, 1,7-Dihydroxy-naphthalin, 2,7-Dihydroxy-naphthalin, 2-Methyl-1-naphthol-acetat, 1,3-Dihydroxy-benzol, 1-Chlor-2,4-dihydroxy-benzol, 1,3-Dihydroxy-2-methyl-benzol, 5-[(2-Hydroxyethyl)amino]-1,3-benzodioxol, 3,4-Diamino-benzoesäure, 3,4-Dihydro-6-hydroxy-1,4(2H)-benzoxazin, 3-Methyl-1-phenyl-5-pyrazolon, 5,6-Dihydroxy-indol, 5,6-Dihydroxy-indolin, 6-Hydroxy-indol, 2,3-Indolindion.

Die Gesamtmenge an direktziehenden Farbstoffen beträgt in dem erfindungsgemäßen Mittel etwa 0,01 bis 7 Gew.%, vorzugsweise etwa 0,2 bis 4 Gew.%. Geignete direktziehende Farbstoffe sind z.B. Triphenylmethanfarbstoffe, aromatische Nitrofarbstoffe, Azofarbstoffe, Chinonfarbstoffe, kationische oder anionische Farbstoffe, insbesondere:

### Nitrofarbstoffe (blau):

1,4-Bis[(2-hydroxyethyl)amino)-2-nitrobenzol, 1-(2-Hydroxyethyl)amino-2-nitro-4-[di(2-hydroxyethyl)amino]-benzol, (HC Blue No. 2), 1-Amino-3-methyl-4-[(2-hydroxyethyl)amino]-6-nitrobenzol, (HC Violet No. 1), 4-[Ethyl-(2-hydroxyethyl)amino]-1-[(2-hydroxyethyl)amino]-2-nitrobenzolhydrochlorid (HC Blue No. 12), 1-[(2,3-Dihydroxypropyl)-amino]-4-[methyl-(2-hydroxyethyl)amino]-2-nitrobenzol (HC Blue No. 10), 1-[(2,3-Dihydroxypropyl)amino]-4-(ethyl-(2-hydroxyethyl)amino]-2-nitrobenzol-hydrochlorid (HC Blue No. 9), 1-(3-Hydroxypropylamino)-4-[di(2-hydroxyethyl)amino]-2-nitrobenzol, (HC Violet No. 2).

### Nitrofarbstoffe (rot):

1-Amino-4-[(2-hydroxyethyl)amino]-2-nitrobenzol (HC Red No. 7), 2-Amino-4,6-dinitro-phenol, 1,4-Diamino-2-nitrobenzol (CI76070), 4-Amino-2-nitro-diphenylamin (HC Red No. 1), 1-Amino-4-[di(2-hydroxyethyl)amino)-2-nitrobenzol-hydrochlorid (HC Red No. 13), 1-Amino-5-chlor-4-[(2-hydroxyethyl)amino]-2-nitrobenzol, 4-Amino-1-[(2-hydroxyethyl)amino]-2-nitrobenzol (HC Red No. 3), 4-Amino-2-nitro-1-((prop-2-en-1-yl)-amino)-benzol, 4-Amino-3-nitrophenol, 4-[(2-Hydroxyethyl)-amino]-3-nitrophenol, 4-[(2-Nitrophenyl)amino]phenol (HC Orange No. 1), 1-[(2-Aminoethyl)amino]-4-(2-hydroxyethoxy)-2-nitrobenzol (HC Orange No. 2), 4-(2,3-Dihydroxypropoxy)-1-[(2-hydroxyethyl)amino]-2-nitrobenzol, (HC Orange No. 3), 1-Amino-5-chlor-4-[(2,3-dihydroxypropyl)amino]-2-nitrobenzol (HC Red No. 10), 5-Chlor-1,4-[di(2,3-dihydroxypropyl)amino] -2-nitrobenzol (HC Red No. 11), 2-[(2-Hydroxyethyl)amino]-4,6-dinitro-phenol, 4-Ethylamino-3-nitrobenzoesäure, 2-[(4-Amino-2-nitrophenyl)amino]-benzoesäure, 2-Chlor-6-ethylamino-4-nitrophenol, 2-Amino-6-chlor-4-nitrophenol, 4-[(3-Hydroxypropyl)amino]-3-nitrophenol, 2,5-Diamino-6-nitropyridin, 3-Amino-6-(methylamino)-2-nitropyridin, 1,2,3,4-Tetrahydro-6-nitrochinoxalin, 7-Amino-3,4-dihydro-6-nitro-2H-1,4-benzoxazin (HC Red No. 14).

### Nitrofarbstoffe (gelb):

1,2-Diamino-4-nitrobenzol (CI76020), 1-Amino-2-[(2-hydroxyethyl)amino]-5-nitrobenzol (HC Yellow No. 5), 1-(2-Hydroxyethoxy)-2-[(2-hydroxyethyl)amino]-5-nitrobenzol, (HC Yellow No. 4), 1-[(2-Hydroxyethyl)amino]-2-nitrobenzol (HC Yellow No. 2), 2-[(2-Hydroxyethyl)amino]-1-methoxy-5-nitrobenzol, 2-Amino-3-nitrophenol, 1-Amino-2-methyl-6-nitrobenzol, 1-(2-Hydroxyethoxy)-3-methylamino-4-nitrobenzol, 2,3-(Dihydroxypropoxy)-3-methylamino-4-nitrobenzol, 2-[(2-Hydroxyethyl)-amino]-5-nitrophenol (HC Yellow No. 11), 3-[(2-Aminoethyl)-amino]-1-methoxy-4-nitrobenzol-hydrochlorid, (HC Yellow No.9), 1-[(2-Ureidoethyl)amino]-4-nitrobenzol, 4-[(2,3-Dihydroxypropyl)amino]-3-nitro-1-trifluormethyl-benzol, (HC Yellow No. 6), 1-Chlor-2,4-bis[(2-hydroxyethyl)amino]-5-nitrobenzol (HC Yellow No. 10), 4-[(2-Hydroxyethyl)amino]-3-nitro-1-methylbenzol, 1-Chlor-4-[(2-hydroxyethyl)amino]-3-nitrobenzol (HC Yellow No. 12), 4-[(2-Hydroxyethyl)amino]-3-nitro-1-trifluormethyl-benzol, (HC Yellow No. 13), 4-[(2-Hydroxyethyl)amino]-3-nitro-benzonitril (HC Yellow No. 14), 4-[(2-Hydroxyethyl)amino]-3-nitro-benzamid (HC Yellow No. 15), 2,4-Dinitro-1-hydroxy-naphthalin.

### Chinonfarbstoffe:

1,4-Di[(2,3-dihydroxypropyl)amino]-9,10-anthrachinon, 1,4-Di[(2-hydroxyethyl)amino]-9,10-anthrachinon (CI61545, Disperse Blue 23), 1-Amino-4-hydroxy-9,10-anthrachinon (CI60710, Disperse Red 15), 1-Hydroxy-4-[(4-methyl-2-sulfophenyl)amino]-9,10-anthrachinon, 7-Beta-D-glucopyranosyl-9,10-dihydro-1-methyl-9,10-dioxo-3,5,6,8-tetrahydroxy-2-anthracencarbonsäure (CI75470, Natural Red 4), 1-[(3-Aminopropyl)amino]-9,10-anthrachinon (HC Red No. 8), 1,4-Diamino-9,10-anthrachinon (CI61100, Disperse Violet No. 1), 1-Amino-4-(methylamino)-9,10-anthrachinon (CI61105, Disperse Violet No. 4, Solvent Violet No. 12), N-(6-((3-Chlor-4-(methylamino)phenyl)imino)-4-methyl-3-oxo-1,4-cyclohexadien-1-yl)harnstoff (HC Red No. 9), 2-((4-(Di(2-hydroxyethyl)amino)phenyl)amino)-5-((2-hydroxyethyl)amino)-2,5-cyclohexadien-1,4-dion (HC Green No. 1), 2-Hydroxy-1,4-naphthochinon (CI75480, Natural Orange No. 6), 1,2-Dihydro-2-(1,3-dihydro-3-oxo-2H-indol-2-yliden)-3H-indol-3-on (CI73000), 1,3-Bis(dicyanomethylen)indan.

### Basische Farbstoffe:

Di[4-(diethylamino)phenyl][4-(ethylamino)naphthyl]carbeniumchlorid (CI42595; Basic Blue No. 7), Di[4-(dimethylamino)-phenyl][4-(phenylamino)naphthyl]carbenium-chlorid (CI44045; Basic Blue No. 26), Basic Blue No. 77, 8-Amino-2-brom-5-hydroxy-4-imino-6-[(3-(trimethylammonio)phenyl)amino]-1(4H)-naphthalinon-chlorid (CI56059; Basic Blue No. 99), Tri(4-amino-3-methylphenyl)carbenium-chlorid (CI42520; Basic Violet No. 2), Di(4-aminophenyl)(4-amino-3-methylphenyl)-carbenium-chlorid (CI42510; Basic Violet No. 14), 1-[(4-Aminophenyl)azo]-7-(trimethylammonio)-2-naphthol-chlorid (CI12250; Basic Brown No. 16), 3-[(4-Amino-2,5-dimethoxyphenyl)azo]-N,N,N-trimethylbenzolaminium-chlorid (CI112605, Basic Orange No. 69), 1-[(4-Amino-2-nitrophenyl)azo]-7-(trimethylammonio)-2-naphthol-chlorid (Basic Brown No. 17), 1-[(4-Amino-3-nitrophenyl)azo]-7-(trimethylammonio)-2-naphthol-chlorid (CI12251; Basic Brown No. 17), 2-((4-Aminophenyl)azo)-1,3-dimethyl-1H-imidazol-3-ium-chlorid (Basic Orange No. 31), 3,7-Diamino-2,8-dimethyl-5-phenylphenazinium-chlorid (CI50240; Basic Red No. 2), 1,4-Dimethyl-5-[(4-(dimethylamino)phenyl)azo]-1,2,4-triazoliumchlorid (CI11055; Basic Red No. 22), 1,3-Dimethyl-2-((4-dimethylamino)phenyl)azo-1H-imidazol-3-ium-chlorid (Basic Red No. 51), 2-Hydroxy-1-[(2-methoxyphenyl)azo]-7-(trimethylammonio)-naphthalin-chlorid (CI12245; Basic Red No. 76), 3-Methyl-1-phenyl-4-[(3-(trimethylammonio)phenyl)azo]-pyrazol-5-on-chlorid (CI12719; Basic Yellow No. 57), 1-Methyl-4-((methyl-phenylhydrazono)methyl)-pyridinium-methylsulfat (Basic Yellow No. 87), 1-(2-Morpholiniumpropylamino)-4-hydroxy-9,10-anthrachinon-methylsulfat, 1-[(3-(Dimethylpropylaminium)propyl)amino]-4-(methylamino)-9,10-anthrachinon-chlorid.

### Neutrale Azofarbstoffe:

1-[Di(2-hydroxyethyl)amino]-3-methyl-4-[(4-nitrophenyl)azo]-benzol (CI11210, Disperse Red No. 17), 1-[Di(2-hydroxyethyl)amino]-4-[(4-nitrophenyl)azo]-benzol (Disperse Black No. 9), 4-[(4-Aminophenyl)azo]-1-(di(2-hydroxyethyl)amino]-3-methylbenzol (HC Yellow No. 7), 2,6-Diamino-3-[(pyridin-3-yl)azo]-pyridin, 2-((4-(Acetylamino)phenyl)azo)-4-methylphenol (CI11855; Disperse Yellow No. 3), Disperse Blue No. 106 (CI111935).

### Saure Farbstoffe:

6-Hydroxy-5-[(4-sulfophenyl)azo]-2-naphthalinsulfonsäuredinatriumsalz (CI15985; Food Yellow No. 3; FD&C Yellow No. 6), 2,4-Dinitro-1-naphthol-7-sulfonsäure-dinatriumsalz (CI10316; Acid Yellow No. 1; Food Yellow No. 1), 2-(Indan-1,3-dion-2-yl)chinolin-x,x-sulfonsäure (Gemisch aus Mono- und Disulfonsäure) (C147005;D&C Yellow No. 10; Food Yellow No. 13, Acid Yellow No. 3), 5-Hydroxy-1-(4-sulfophenyl)-4-[(4-sulfophenyl)azo]pyrazol-3-carbonsäure-trinatriumsalz (CI19140; Food Yellow No. 4; Acid Yellow No. 23), 5-[(2,4-Dinitrophenyl)amino]-2-phenylamino-benzolsulfonsäurenatriumsalz (CI10385; Acid Orange No. 3), 4-[(2-Hydroxynaphth-1-yl)azo]-benzolsulfonsäure-natriumsalz (CI15510; Acid Orange No. 7), 4-((2-Hydroxy-naphthalin-1-yl)azo)-3-methyl-benzolsulfonsäure-natriumsalz (CI15575; Acid Orange No. 8), 3',6'-Dihydroxy-4',5'-diiodospiro(isobenzofuran-1(3H)-9'-(9H)xanthen)-3-on (CI45425, D&C Orange No. 10), 4-Hydroxy-3-[(2-methoxyphenyl)azo]-1-naphthalinsulfonsäuremononatriumsalz (CI14710; Acid Red No. 4), 6-Hydroxy-5-[(4-sulfonaphth-1-yl)azo]-2,4-naphthalin-disulfonsäure-trinatriumsalz (CI16255; Ponceau 4R; Acid Red No. 18), 8-Amino-1-hydroxy-2-(phenylazo)-3,6-naphthalin-disulfonsäuredinatriumsalz (CI17200; Acid Red No. 33), 5-(Acetylamino)-4-hydroxy-3-[(2-methylphenyl)azo]-2,7-naphthalin-disulfonsäure-dinatriumsalz (CI18065; Acid Red No. 35), N-[6-(Diethylamino)-9-(2,4-disulfophenyl)-3H-xanthen-3-yliden]-N-ethylethanammonium-hydroxid, inneres Salz, Natriumsalz (CI45100; Acid Red No. 52), 2',4',5',7'-Tetrabrom-3',6'-dihydroxyspiro[isobenzofuran-1(3H),9'-[9H]xanthen]-3-ondinatriumsalz (CI45380; Acid Red No. 87), 2',4',5',7'-Tetrabrom-4,5,6,7-tetrachlor-3',6'-dihydroxyspiro[isobenzofuran-1(3H),9'[9H]xanthen]-3-on-dinatriumsalz (CI45410; Acid Red No. 92), 3',6'-Dihydroxy-4',5'-diiodospiro[isobenzofuran-1(3H), 9'(9H)-xanthen]-3-on-dinatriumsalz (CI45425; Acid Red No. 95), 2-Hydroxy-3-((2-hydroxynaphth-1-yl)azo)-5-nitrobenzolsulfonsäure-mononatriumsalz (CI15685; Acid Red No. 184), (2-Sulfophenyl)di[4-(ethyl((4-sulfophenyl)methyl)amino)phenyl]-carbenium-dinatriumsalz, betain (CI42090; Acid Blue No. 9; FD&C Blue No. 1), 3-Hydroxy-4-((4-methyl-2-sulfophenyl)azo)-2-naphthalincarbonsäuredinatriumsalz (CI15850; D&C Red No. 6), 6-Hydroxy-5-((2-methoxy-5-methyl-4-sulfophenyl)azo)-2-naphthalinsulfonsäuredinatriumsalz (CI16035; FD&C Red 40), 1,4-Bis[(2-sulfo-4-methylphenyl)amino]-9,10-anthrachinon-dinatriumsalz (CI 61570; Acid Green No. 25), 1-Amino-4-(cyclohexylamino)-9,10-anthrachinon-2-sulfonsäure-natriumsalz (CI62045; Acid Blue No. 62), 3,3-Bis(3,5-dibrom-4-hydroxyphenyl)-4,5,6,7-tetrabrom-2,1(3h)-benzoxathiol-1,1-dioxid, 1-Amino-4-(phenylamino)-9,10-anthrachinon-2-sulfonsäure (CI62055; Acid Blue No. 25), 1-Hydroxy-4-[(4-methyl-2-sulfophenyl)-amino]-9,10-anthrachinon-natriumsalz (CI60730; D&C Violet No. 2; Acid Violet No. 43), 5-Amino-4-hydroxy-6-[(4-nitrophenyl)azo]-3-(phenylazo)-2,7-naphthalin-disulfonsäuredinatriumsalz (CI20470; Acid Black No. 1), 3-Hydroxy-4-[(2-hydroxynaphth-1-yl)azo]-7-nitro-1-naphthalin-sulfonsäurechromkomplex (3:2) (CI15711; Acid Black No. 52), 3-[(2,4-Dimethyl-5-sulfophenyl)azo]-4-hydroxy-1-naphthalin-sulfonsäure-dinatriumsalz (CI14700; Food Red No.1; FD&C Red No.4).

Weitere zur Haarfärbung bekannte und übliche Farbstoffe, die in dem erfindungsgemäßen Färbemittel enthalten sein können, sind unter anderem in E. Sagarin, "Cosmetics, Science and Technology", Interscience Publishers Inc., New York (1957), Seiten 503 ff. sowie H. Janistyn, "Handbuch der Kosmetika und Riechstoffe", Band 3 (1973), Seiten 388 ff. und K. Schrader "Grundlagen und Rezepturen der Kosmetika", 2. Auflage (1989), Seiten 782-815 beschrieben.

Für die Anwendung zur oxidativen Färbung vermischt man ein erfindungsgemäßes Färbemittel unmittelbar vor dem Gebrauch mit einer Oxidationsmittelzusammensetzung und trägt eine für die Färbung ausreichende Menge der gebrauchsfertigen Zubereitung auf die Haare auf. Sofern das erfindungsgemäße Färbemittel keine Oxidationsfarbstoffvorstufen enthält beziehungsweise solche Oxidationsfarbstoffvorstufen enthält, welche mit Luftsauerstoff leicht oxidierbar sind, kann es ohne vorheriges Vermischen mit einem Oxidationsmittel direkt auf die Keratinfaser aufgetragen werden. Als Oxidationsmittel zur Entwicklung der Färbung kommen hauptsächlich Wasserstoffperoxid oder dessen Additionsverbindungen an Harnstoff, Melamin oder Natriumborat in Form einer 1 bis 12%igen, vorzugsweise einer 1,5 bis 6%igen wässrigen Lösung in Betracht. Das Mischungsverhältnis von Färbemittel zu Oxidationsmittel ist abhängig von der Konzentration des Oxidationsmittels und beträgt in der Regel etwa 5:1 bis 1:2, vorzugsweise 1:1, wobei der Gehalt an Oxidationsmittel in der gebrauchsfertigen Zubereitung vorzugsweise etwa 0,5 bis 8 Gew.%, insbesondere 1 bis 4 Gew.% beträgt.

Das erfindungsgemäße Mittel wird bevorzugt in einem wässrigen, einem alkoholischen oder in einem wässrig-alkoholischen Medium mit vorzugsweise mindestens 10 Gew.% Wasser konfektioniert. Als Alkohole können insbesondere die für kosmetische Zwecke üblicherweise verwendeten niederen Alkohole mit 1 bis 4 C-Atomen wie z.B. Ethanol und Isopropanol enthalten sein. Das erfindungsgemäße Mittel kann in einem pH-Bereich von 2,0 bis 9,5 vorliegen. Besonders bevorzugt ist der pH-Bereich zwischen 4 und 8. Als zusätzliche Co-Solventien können organische Lösungsmittel oder ein Gemisch aus Lösungsmitteln mit einem Siedepunkt unter 400°C in einer Menge von 0,1 bis 15 Gew.% bevorzugt von 1 bis 10 Gew.% enthalten sein. Besonders geeignet als zusätzliche Co-Solventien sind unverzweigte oder verzweigte Kohlenwasserstoffe wie Pentan, Hexan, Isopentan und cyclische Kohlenwasserstoffe wie Cyclopentan und Cyclohexan. Weitere, besonders bevorzugte wasserlösliche Lösungsmittel sind Glycerin, Ethylenglykol und Propylenglykol in einer Menge bis 30 Gew.%.

Das erfindungsgemäße Mittel kann darüber hinaus weitere, für Haarbehandlungsmittel übliche Zusatzbestandteile enthalten, z.B. Parfümöle; Trübungsmittel wie z.B. Ethylenglykoldistearat, Styrol/PVP Copolymere oder Polystyrole; Feuchthaltemittel; partikelförmige Stoffe; Konservierungsmittel; Glanzgeber; Produktanfärbestoffe; Antioxidantien; in Mengen von jeweils vorzugsweise 0,01 bis 10 Gew.%, wobei die Gesamtmenge vorzugsweise 10 Gew.% nicht übersteigt.

Das erfindungsgemäße Mittel kann in verschiedenen Applikationsformen Anwendung finden, z.B. in Form von Haarpflege-, Haarfestigungs-, Haarreinigungs-, Haarfärbe- oder Tönungsmittel, als Blondiermittel oder als Dauerwellmittel. Das Mittel kann als Lotion, Aerosol-Schaum, Non-Aerosol Schaum, Haarmilch, Haargel, Flüssiggel, Sprühgel, Haarcreme, Gelschaum, Aerosol- oder Nonaerosolspray, Haarwachs oder in Form eines emulsionsförmigen Haarpflegemittels (Haarspülung, Conditioner) appliziert werden. Auch ein Einsatz in Form einer mit einem üblichen Verdicker verdickten Lotion ist möglich. Das Mittel kann auch als Shampoo in Kombination mit vorzugsweise anionischen, amphoteren und nichtionischen Tensiden oder als Färbemittel in Kombination mit direktziehenden oder oxidativen Haarfarben, z.B. als Farbfestiger vorliegen.

In einer Ausführungsform liegt das erfindungsgemäße Mittel in Form eines Gels, in Form einer viskosen Lotion oder in Form eines Sprühgels, welches mit einer mechanischen Vorrichtung versprüht wird, vor und enthält mindestens ein verdickendes Polymer in einer Menge von vorzugsweise 0,05 bis 10, besonders bevorzugt von 0,1 bis 2 Gew.% und weist eine Viskosität von mindestens 250 mPa s (gemessen mit einem Bohlin Rheometer CS, Messkörper C25 bei 25°C und einer Schergeschwindigkeit von 50 s⁻¹) auf. Die Viskosität des Gels beträgt vorzugsweise von 500 bis 50.000 mPa s, besonders bevorzugt von 1.000 bis 15.000 mPa s bei 25°C. Die verdickenden Polymere können ausgewählt sein z.B. aus Copolymeren aus mindestens einer ersten Monomerart, die ausgewählt ist aus Acrylsäure und Methacrylsäure und mindestens einer zweiten Monomerart, die ausgewählt ist aus Estern der Acrylsäure und ethoxyliertem Fettalkohol; vernetzter Polyacrylsäure; vernetzte Copolymere aus mindestens einer ersten Monomerart, die ausgewählt ist aus Acrylsäure und Methacrylsäure und mindestens einer zweiten Monomerart, die ausgewählt ist aus Estern der Acrylsäure mit C10- bis C30-Alkoholen; Copolymeren aus mindestens einer ersten Monomerart, die ausgewählt ist aus Acrylsäure und Methacrylsäure und mindestens einer zweiten Monomerart, die ausgewählt ist aus Estern der Itaconsäure und ethoxyliertem Fettalkohol; Copolymeren aus mindestens einer ersten Monomerart, die ausgewählt ist aus Acrylsäure und Methacrylsäure, mindestens einer zweiten Monomerart, die ausgewählt ist aus Estern der Itaconsäure und ethoxyliertem C10- bis C30-Alkohol und einer dritten Monomerart, ausgewählt aus C1- bis C4-Aminoalkylacrylaten; Copolymeren aus zwei oder mehr Monomeren, ausgewählt aus Acrylsäure, Methacrylsäure, Acrylsäureestern und Methacrylsäureestern; Copolymeren aus Vinylpyrrolidon und Ammoniumacryloyldimethyltaurat; Copolymeren aus Ammoniumacryloyldimethyltaurat und Monomeren ausgewählt aus Estern von Methacrylsäure und ethoxylierten Fettalkoholen; Hydroxyethylcellulose; Hydroxypropylcellulose; Hydroxypropylguar; Glycerylpolyacrylat; Glycerylpolymethacrylat; Copolymeren aus mindestens einem C2- C3- oder C4-Alkylen und Styrol; Polyurethanen; Hydroxypropylstärkephosphat; Polyacrylamid; mit Decadien vernetztes Copolymer aus Maleinsäureanhydrid und Methylvinylether; Johannesbrotkernmehl; Guar-Gummi; Xanthan; Dehydroxanthan; Carrageenan; Karaya-Gummi; hydrolysierte Maisstärke; Copolymere aus Polyethylenoxid, Fettalkoholen und gesättigtem Methylendiphenyldiisocyanat (z.B. PEG-150/Stearylalkohol/ SMDI Copolymer).

Eine spezielle Ausführungsform stellen Aerosol-Sprühgele dar. Die Verdickung wird durch die Wechselwirkung des in wässrig-ethanolischem Medium gelösten Chitosanderivates mit dem Treibgas, z.B. Dimethylether erreicht. Man erhält eine Gelee-artige Masse, die sich aus Aerosolflaschen aus z.B. Glas oder Metall leicht versprühen läßt.

In einer Ausführungsform liegt das erfindungsgemäße Mittel in Form einer O/W-Emulsion, einer W/O-Emulsion oder einer Mikroemulsion vor und und enthält mindestens eines der oben genannten, in Wasser emulgierten Öle oder Wachse sowie mindestens eines der obengenannten Tenside.

In einer Ausführungsform liegt das erfindungsgemäße Mittel in Form eines Sprühproduktes vor, entweder in Kombination mit einer mechanischen Pumpsprühvorrichtung oder in Kombination mit mindestens einem Treibmittel, ausgewählt aus Propan, Butan, Dimethylether und fluorierten Kohlenwasserstoffen. Ein Aerosolsprays enthält zusätzlich vorzugsweise 15 bis 85 Gew.%, besonders bevorzugt 25 bis 75 Gew.% eines Treibmittels und wird in einem Druckbehälter abgefüllt. Als Treibmittel sind beispielsweise niedere Alkane, wie z.B. n-Butan, i-Butan und Propan, oder auch deren Gemische sowie Dimethylether oder Fluorkohlenwasserstoffe wie F 152a (1,1-Difluorethan) oder F 134 (Tetrafluorethan) sowie ferner bei den in Betracht kommenden Drücken gasförmig vorliegende Treibmittel, wie beispielsweise N₂, N₂O und CO₂ sowie Gemische der vorstehend genannten Treibmittel geeignet.
Ein Non-Aerosol-Haarsprays wird mit Hilfe einer geeigneten mechanisch betriebenen Sprühvorrichtung versprüht. Unter mechanischen Sprühvorrichtungen sind solche Vorrichtungen zu verstehen, welche das Versprühen einer Zusammensetzung ohne Verwendung eines Treibmittels ermöglichen. Als geeignete mechanische Sprühvorrichtung kann beispielsweise eine Sprühpumpe oder ein mit einem Sprühventil versehener elastischer Behälter, in dem das erfindungsgemäße kosmetische Mittel unter Druck abgefüllt wird, wobei sich der elastische Behälter ausdehnt und aus dem das Mittel infolge der Kontraktion des elastischen Behälters bei Öffnen des Sprühventils kontinuierlich abgegeben wird, verwendet werden.

In einer Ausführungsform liegt das erfindungsgemäße Mittel in Form eines verschäumbaren Produktes (Mousse) in Kombination mit einer Vorrichtungen zum Verschäumen vor, enthält mindestens eine übliche, hierfür bekannte schaumgebende Substanz, z.B. mindestens ein schaumbildendes Tensid oder mindestens ein schaumbildendes Polymer. Unter Vorrichtungen zum Verschäumen sind solche Vorrichtungen zu verstehen, welche das Verschäumen einer Flüssigkeit mit oder ohne Verwendung eines Treibmittels ermöglichen. Als geeignete mechanische Schäumvorrichtung kann beispielsweise ein handelsüblicher Pumpschäumer oder ein Aerosolschaumkopf verwendet werden. Das Produkt liegt entweder in Kombination mit einer mechanischen Pumpschäumvorrichtung (Pumpschaum) oder in Kombination mit mindestens einem Treibmittel (Aerosolschaum) in einer Menge von vorzugsweise 1 bis 20, insbesondere von 2 bis 10 Gew.%, vor. Treibmittel sind z.B. ausgewählt aus Propan, Butan, Dimethylether und fluorierten Kohlenwasserstoffen. Das Mittel wird unmittelbar vor der Anwendung verschäumt und als Schaum in das Haar eingearbeitet und kann anschließend ausgespült werden oder ohne Ausspülen im Haar belassen werden.

In einer Ausführungsform liegt das erfindungsgemäße Mittel in Form eines Haarwachses vor, d.h. es weist wachsartige Konsistenz auf und enthält mindestens einen der oben genannten Wachse in einer Menge von vorzugsweise 0,5 bis 30 Gew.% sowie gegebenenfalls weitere wasserunlösliche Stoffe. Die wachsartige Konsistenz ist vorzugsweise dadurch gekennzeichnet, dass die Nadelpenetrationszahl (Maßeinheit 0,1 mm, Prüfgewicht 100 g, Prüfdauer 5 s, Prüftemperatur 25°C; nach DIN 51 579) größer oder gleich 10, besonders bevorzugt größer oder gleich 20 ist und dass der Erstarrungspunkt des Produktes vorzugsweise größer oder gleich 30°C und kleiner oder gleich 70°C ist, besonders bevorzugt im Bereich von 40 bis 55°C liegt. Geeignete Wachse und wasserunlösliche Stoffe sind insbesondere Emulgatoren mit einem HLB-Wert unterhalb von 7, Silikonöle, Silikonwachse, Wachse (z.B. Wachsalkohole, Wachssäuren, Wachsester, sowie insbesondere natürliche Wachse wie Bienenwachs, Carnaubawachs, etc.), Fettalkohole, Fettsäuren, Fettsäureester oder hydrophile Wachse wie z.B. hochmolekulare Polyethylenglykole mit einem Molekulargewicht von 800 bis 20.000, vorzugsweise von 2.000 bis 10.000 g/mol.

Wenn das erfindungsgemäße Haarbehandlungsmittel in Form einer Haarlotion vorliegt, so liegt es als im wesentlichen nicht-viskose oder gering viskose, fließfähige Lösung, Dispersion oder Emulsion mit einem Gehalt an mindestens 10 Gew.%, vorzugsweise 20 bis 95 Gew.% eines kosmetisch verträglichen Alkohols vor. Als Alkohole können insbesondere die für kosmetische Zwecke üblicherweise verwendeten niederen Alkohole mit 1 bis 4 C-Atomen, z.B. Ethanol und Isopropanol verwendet werden.

Wenn das erfindungsgemäße Haarbehandlungsmittel in Form einer Haarcreme vorliegt, so liegt es vorzugsweise als Emulsion vor und enthält entweder zusätzlich viskositätsgebende Inhaltsstoffe in einer Menge von 0,1 bis 10 Gew.% oder die erforderliche Viskosität und cremige Konsistenz wird durch Micellbildung mit Hilfe von geeigneten Emulgatoren, Fettsäuren, Fettalkoholen, Wachsen etc. in üblicher Weise aufgebaut.

Wenn das erfindungsgemäße Haarbehandlungsmittel in Form eines Haarreinigungsmittels vorliegt, so enthält es zusätzlich mindestens ein waschaktives Tensid, vorzugsweise 0,01 bis 25 Gew.%, insbesondere 5 bis 20 Gew.% mindestens eines anionischen, amphoteren und/oder nicht-ionischen Tensids und 50 bis 90 Gew.% Wasser. Das erfindungsgemässe Haarreinigungsmittel weist einen pH-Wert von vorzugsweise 3 bis 8, insbesondere von 4 bis 7 auf. Geeignete Tenside sind z.B. die oben genannten. Bevorzugte Tenside für ein erfindungsgemäßes Haarreinigungsmittel sind ausgewählt aus Alkali- oder Erdalkalisalzen der C10- bis C18-Alkylsulfate, der C10- bis C18-Alkylsulfonate, der C10- bis C18-Alkylbenzolsulfonate, der C10- bis C18-Xylolsulfonate und der mit 1 bis 10 Ethylenoxideinheiten ethoxylierten C10- bis C18-Alkylethersulfate; den ethoxylierten Sulfobernsteinsäurehalbestern der allgemeinen Formel R¹(OCH₂CH₂)ₘ-O₂C-CH₂CH(SO₃M)-CO₂M, wobei R1 einen C10- bis C18-Alkylrest bedeutet, M ein Alkali- oder Erdalkalikation darstellt und m eine ganze Zahl von 1 bis 10 bedeutet; den Alkylethercarboxylaten der Formel R²(OCH₂CH₂)ₙ-OCH₂COOM, wobei R2 einen C10 bis C18-Alkylrest bedeutet, M ein Alkali- oder Erdalkalikation darstellt und n eine ganze Zahl von 1 bis 20 bedeutet; ethoxylierten Fettalkoholen mit 12 bis 18 C-Atomen; Polyglycerylethern von gesättigten oder ungesättigten Fettalkoholen und Alkylphenolen mit 8 bis 30 C-Atomen im Alkylrest und 1 bis 10 Glyceryleinheiten im Molekül; Fettsäurealkanolamiden; ethoxylierten Sorbitanfettsäureestern, C8-C18-Alkylpolyglucosiden, C8- bis C18-Alkylbetainen und amphoteren Tensiden der Formel 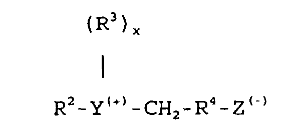 wobei R2 eine gerad- oder verzweigtkettige Alkyl-, Alkenyl- oder Hydroxyalkylgruppe mit 8 bis 18 C-Atomen und 0 bis 10 Ethylenoxideinheiten und 0 bis 1 Glycerineinheit darstellt; Y ein N-, P- oder S-Atom ist; R3 eine Alkyl- oder Monohydroxyalkylgruppe mit 1 bis 3 C-Atomen ist; x gleich 1 ist, falls Y ein Schwefelatom ist und x gleich 2 ist, wenn Y ein Stickstoff- oder Phosphoratom ist; R4 eine Alkylen- oder Hydroxyalkylengruppe mit 1 bis 4 C-Atomen ist und Z eine Carboxylat-, Sulfat-, Phosphonat- oder Phosphatgruppe darstellt.

Gegenstand der Erfindung auch ein Verfahren zur Haarbehandlung, wobei
- ein erfindungsgemäßes Haarbehandlungsmittel bereit gestellt wird,
- das Haarbehandlungsmittel auf das Haar aufgebracht wird und
- das Haarbehandlungsmittel entweder nach einer Einwirkszeit aus dem Haar ausgepült oder im Haar belassen wird.

Wird das erfindungsgemäße kosmetische Mittel zur Haarpflege oder -festigung eingesetzt, so wird es folgendermaßen angewandt: Nach der Haarwäsche werden in dem handtuchtrockenen Haar, je nach Haarfülle, 5 bis 30 g des Mittels verteilt. Anschließend wird das Haar durchgekämmt und zur Frisur geformt und getrocknet.

Die nachfolgenden Beispiele sollen den Gegenstand der Erfindung näher erläutern. Die angegebenen Polymergehalte beziehen sich, wenn nicht anders angegeben, jeweils auf den Feststoffgehalt. Prozentzahlen stellen Gewichtsprozente dar, sofern nichts anderes vermerkt ist.

### Beispiele

Das in den Beispielen eingesetzte N-Hydroxypropyl-O-benzylchitosan (HPBC) ist herstellbar analog dem in der EP 0 300 234 beschriebenen Verfahren durch Umsetzung von Chitosan mit einer Molmasse zwischen 300.000 und 700.000 und einem Acetylierungsgrad zwischen 0,1 und 0,3 mit 2 bis 7 Äquivalenten Propylenoxid und 0,25 bis 1,5 Äquivalenten Benzylchlorid.

### Beispiel 1: auszuspülender Haarpflegeschaum

| | A | B | C | D | V |
|---|---|---|---|---|---|
| HPBC | 0,50 | 1,50 | 2,50 | 4,00 | - |
| PEG-35 caster oil | 0,3 | 0,3 | 0,3 | 0,3 | 0,3 |
| PEG-4-laurylether | 0,3 | 0,3 | 0,3 | 0,3 | 0,3 |
| Cetrimoniumchlorid | 0,1 | 0,1 | 0,1 | 0,1 | 0,1 |
| Isopropanol | 0,07 | 0,07 | 0,07 | 0,07 | 0,07 |
| Ethanol | 10 | 10 | 10 | 10 | 10 |
| Polyquaternium-16¹⁾ | 1,4 | 1,4 | 1,4 | 1,4 | 1,4 |
| Parfümöl | 0,3 | 0,3 | 0,3 | 0,3 | 0,3 |
| Wasser | ad 100 | ad 100 | ad 100 | ad 100 | ad 100 |

| | | | | | |
|---|---|---|---|---|---|
| ¹⁾ Vinylimidazoliminiummethochlorid/Vinylpyrrolidon Copolymer | | | | | |

96 g des Haarpflegemittels werden mit 4 g eines Gemisches aus 50 Gew.% Propan und 50 Gew.% n-Butan in einen Aerosolbehälter abgefüllt.Der erfindungsgemässe Pflegeschaum wird beim Versprühen in Schaumform dem Behälter entnommen.

Die erfindungsgemäßen Zusammensetzungen A bis D zeichneten sich gegenüber der Vergleichszusammensetzung V aus durch
- Erhöhte Elastizität der Frisur
- Erhöhtes Volumen der Frisur
- Besserer Griff und bessere Kämmbarkeit des feuchten und trockenen Haares

### Beispiel 2: Volumenshampoo

| | A | B | C | D | V |
|---|---|---|---|---|---|
| HPBC | 0,50 | 1,50 | 2,50 | 4,00 | - |
| Natriumlaurylethersulfat | 2,7 | 2,7 | 2,7 | 2,7 | 2,7 |
| Benzoesäure | 0,03 | 0,03 | 0,03 | 0,03 | 0,03 |
| Kokosfettsäureamidopropylbetain | 1,5 | 1,5 | 1,5 | 1,5 | 1,5 |
| Ethylenglykoldistearat | 2 | 2 | 2 | 2 | 2 |
| Parfümöl | 0,4 | 0,4 | 0,4 | 0,4 | 0,4 |
| Natriumchlorid | 3,3 | 3,3 | 3,3 | 3,3 | 3,3 |
| Wasser | ad 100 | ad 100 | ad 100 | ad 100 | ad 100 |

Die erfindungsgemäßen Zusammensetzungen A bis D zeichneten sich gegenüber der Vergleichszusammensetzung V aus durch
- Erhöhte Elastizität der Frisur
- Erhöhtes Volumen der Frisur
- Besserer Griff und bessere Kämmbarkeit des feuchten und trockenen Haares

### Beispiel 3: Haarstylingschaum

| | A | B | C | D | V |
|---|---|---|---|---|---|
| HPBC | 0,50 | 1,50 | 2,50 | 4,00 | 0,00 |
| Polyquaternium-11 | 6,5 | 4,5 | 3,0 | 2,5 | 8 |
| Cetrimoniumchlorid | 0,1 | 0,1 | 0,75 | 0,5 | 0,2 |
| Ethanol | 0,5 | 0,5 | 0,5 | 0,5 | 0,5 |
| Wasser | ad 100 | ad 100 | ad 100 | ad 100 | ad 100 |

Die erfindungsgemäßen Zusammensetzungen A bis D zeichneten sich gegenüber der Vergleichszusammensetzung V aus durch
- Verringerte Belastung
- Verbesserter Glanz
- Verbesserte Festigung

### Beispiel 4: Haar-Spraygel

| | A | B | C | D | V |
|---|---|---|---|---|---|
| HPBC | 0,1 | 0,5 | 1,5 | 2,5 | - |
| Acrylates Copolymer | 2,00 | 2,10 | - | - | 2,50 |
| Carbomer | - | - | 0,08 | 0,10 | - |
| Aminomethylpropanol | 0,17 | 0,15 | 0,08 | 0,11 | 0,18 |
| Glycerin | 0,10 | 0,10 | 0,10 | 0,10 | 0,10 |
| PEG-40 Hydrogenated Castor Oil | 0,20 | 0,20 | 0,20 | 0,20 | 0,20 |
| Parfüm | 0,20 | 0,20 | 0,20 | 0,20 | 0,20 |
| Ethanol | 25,00 | 25,00 | 25,00 | 25,00 | 25,00 |
| Polyvinylpyrrolidon/ Vinylacetat Copolymer | 2,50 | 5,00 | 2,50 | 3,20 | 0,00 |
| Polyvinylpyrrolidon | 0,00 | 0,00 | 0,00 | 0,00 | 2,60 |
| Wasser | ad 100 | Ad 100 | ad 100 | ad 100 | ad 100 |

### Beispiel 5: Haarbalsam

| | A | B | C | D |
|---|---|---|---|---|
| HPBC | 0,50 | 1,50 | 2,50 | 4,00 |
| Glycerylstearat/Polyethylen glykol-(20)-cetearylether | 6 | 6 | 6 | 6 |
| Diquaternäres Polydimethylsiloxan (Abil® Quat 3272) | 4 | 4 | 4 | 4 |
| Cetylalkohol | 2 | 2 | 2 | 2 |
| Zitronensäure | 1,36 | 1,36 | 1,36 | 1,36 |
| Konservierung, Parfüm | q.s. | q.s. | q.s. | q.s. |
| Wasser | ad 100 | ad 100 | ad 100 | ad 100 |

### Beispiel 6: Haarspülung

| | A | B | C | D |
|---|---|---|---|---|
| HPBC | 0,50 | 1,50 | 2,50 | 4,00 |
| Cetylstearylalkohol | 4 | 4 | 4 | 4 |
| Pyrrolidoncarbonsäure | 1,36 | 1,36 | 1,36 | 1,36 |
| Cetyltrimethylammoniumchlorid | 0,75 | 0,75 | 0,75 | 0,75 |
| Parfüm | 0,5 | 0,5 | 0,5 | 0,5 |
| Pflanzenextrakt Extrapon® 5 Spezial | 0,2 | 0,2 | 0,2 | 0,2 |
| Wasser | ad 100 | ad 100 | ad 100 | ad 100 |

### Beispiel 7: Versprühbares Haarkurmittel

| | A | B | C | D |
|---|---|---|---|---|
| HPBC | 0,50 | 1,50 | 2,50 | 4,00 |
| Poly(dimethyldiallylammoniumchlorid) | 2 | 2 | 2 | 2 |
| Polyethylenglykol-(40)-sorbitanmonopalmitat | 1,25 | 1,25 | 1,25 | 1,25 |
| Pyrrolidoncarbonsäure | 1 | 1 | 1 | 1 |
| Parfüm | 0,1 | 0,1 | 0,1 | 0,1 |
| Cetyltrimethylammoniumchlorid | 0,03 | 0,03 | 0,03 | 0,03 |
| Ethanol | 15 | 15 | 15 | 15 |
| Wasser | ad 100 | ad 100 | ad 100 | ad 100 |

### Beispiel 8: Schaumförmiges Haarkurmittel

| | A | B | C | D |
|---|---|---|---|---|
| HPBC | 0,50 | 1,50 | 2,50 | 4,00 |
| Dow Corning 929 (Amodimethicone, 35%ig) | 2 | 2 | 2 | 2 |
| Zitronensäure | 1,3 | 1,3 | 1,3 | 1,3 |
| Hydroxypropylcellulose (M = 1.150.000 g/mol) | 0,5 | 0,5 | 0,5 | 0,5 |
| Dow Corning 2501 Cosmetic Wax¹⁾ | 0,3 | 0,3 | 0,3 | 0,3 |
| Parfüm | 0,2 | 0,2 | 0,2 | 0,2 |
| Cetyltrimethylammoniumchlorid | 0,25 | 0,25 | 0,25 | 0,25 |
| D-Panthenol | 0,15 | 0,15 | 0,15 | 0,15 |
| Elastinhydrolysat | 0,1 | 0,1 | 0,1 | 0,1 |
| Propan/Butan (5,0 bar) | 5 | 5 | 5 | 5 |
| Ethanol | 10 | 10 | 10 | 10 |
| Wasser | ad 100 | ad 100 | ad 100 | ad 100 |

| | | | | |
|---|---|---|---|---|
| ¹⁾ BIS-PEG-18 METHYL ETHER DIMETHYL SILANE | | | | |

### Beispiel 9: Haarfestigungsmittel

| | A | B | C | D |
|---|---|---|---|---|
| HPBC | 0,1 | 0,5 | 1,5 | 2,5 |
| Vinylpyrrolidon/Vinylacetat Copolymer | 3 | 3 | 3 | 3 |
| 1,2-Propylenglykol | 0,2 | 0,2 | 0,2 | 0,2 |
| Parfüm | 0,15 | 0,15 | 0,15 | 0,15 |
| Cetyltrimethylammoniumchlorid | | | | |
| Wasser | 20 | 20 | 20 | 20 |
| Ethanol | ad 100 | ad 100 | ad 100 | ad 100 |

### Beispiel 10: Schaumfestiger

| | A | B | C | D |
|---|---|---|---|---|
| HPBC | 0,1 | 0,5 | 1,5 | 2,5 |
| Polyvinylpyrrolidon | 3,15 | 3,15 | 3,15 | 3,15 |
| Zitronensäure | 1,6 | 1,6 | 1,6 | 1,6 |
| Hydriertes Rizinusöl, ethoxyliert mit 40 Mol Ethylenoxid | 0,6 | 0,6 | 0,6 | 0,6 |
| Decylpolyglucosid | 0,22 | 0,22 | 0,22 | 0,22 |
| Vinylpyrrolidon/Methacrylamidopropyltrimethylammoniumchlorid Copolymer | 0,2 | 0,2 | 0,2 | 0,2 |
| Parfüm | 0,2 | 0,2 | 0,2 | 0,2 |
| Propan/Butan (5,0 bar) | 6 | 6 | 6 | 6 |
| Wasser | ad 100 | ad 100 | ad 100 | ad 100 |

### Beispiel 11: Haarspray

| | A | B | C | D |
|---|---|---|---|---|
| HPBC | 0,1 | 0,5 | 1,5 | 2,5 |
| Acrylates/Acrylamide Copolymer | 3,5 | 3,5 | 3,5 | 3,5 |
| Wasser | 3,5 | 5 | 6 | 10 |
| Parfüm | 0,15 | 0,15 | 0,15 | 0,15 |
| Dimethylether | 45 | 45 | 45 | 45 |
| Ethanol | ad 100 | ad 100 | ad 100 | ad 100 |

### Beispiel 12: 80% VOC-Haarspray

| | A | B | C | D |
|---|---|---|---|---|
| HPBC | 0,1 | 0,5 | 1,5 | 2,5 |
| t-Butylacrylat/Ethylacrylat/ Methacrylsäure Terpolymer | 4 | 4 | 4 | 4 |
| 2-Amino-2-methyl-1-propanol | 0,72 | 0,72 | 0,72 | 0,72 |
| Cyclo-tetra(dimethylsiloxan) | 0,2 | 0,2 | 0,2 | 0,2 |
| Parfüm | 0,05 | 0,05 | 0,05 | 0,05 |
| Wasser | 15 | 15 | 15 | 15 |
| Dimethylether | 40 | 40 | 40 | 40 |
| Ethanol | ad 100 | ad 100 | ad 100 | ad 100 |

### Beispiel 13: Pump-Haarspray

| | A | B | C | D |
|---|---|---|---|---|
| HPBC | 0,1 | 0,5 | 1,5 | 3,5 |
| Vinylpyrrolidon/Vinylacetat Copolymer | 4 | 4 | 4 | 4 |
| Ethanol | 28,5 | 28,5 | 28,5 | 28,5 |
| Parfüm | 0,25 | 0,25 | 0,25 | 0,25 |
| Cetyltrimethylammoniumbromid | 0,2 | 0,2 | 0,2 | 0,2 |
| Wasser | ad 100 | ad 100 | ad 100 | ad 100 |

### Beispiel 14: Farbfestiger

| | A | B | C |
|---|---|---|---|
| HPBC | 0,50 | 1,50 | 2,50 |
| Vinylpyrrolidon/Vinylacetat Copolymer | 3,5 | 3,5 | 3,5 |
| Parfüm | 0,2, | 0,2 | 0,2 |
| 1-Amino-4-(2',3'-dehydroxypropyl)amino-5-chlor-2-nitrobenzol | 0,07 | 0,07 | 0,07 |
| Basic Brown 17 (C. I. 12 251) | 0,05 | 0,05 | 0,05 |
| Basic Blue 7 (C. I. 42 595) | 0,01 | 0,01 | 0,01 |
| Basic Violett 14 (C. I. 42 510) | 0,0023 | 0,0023 | 0,0023 |
| Ethanol | 50 | 50 | 50 |
| Wasser | ad 100 | ad 100 | ad 100 |

### Beispiel 15: Haarpflegespülung

| | A | B | C | D |
|---|---|---|---|---|
| HPBC | 0,50 | 1,50 | 2,50 | 4,00 |
| Cetylstearylalkohol | 2,5 | 2,5 | 2,5 | 2,5 |
| Laurylalkoholdiglykolether | 0,8 | 0,8 | 0,8 | 0,8 |
| Vaseline | 1,1 | 1,1 | 1,1 | 1,1 |
| Cetylstearylsulfat-Natriumsalz | 0,4 | 0,4 | 0,4 | 0,4 |
| Betainmonohydrat | 5 | 5 | 5 | 5 |
| Parfümöl | 0,2 | 0,2 | 0,2 | 0,2 |
| Wasser | ad 100 | ad 100 | ad 100 | ad 100 |

### Beispiel 16: Haarpflegemittel, nicht auszuspülen

| | A | B | C |
|---|---|---|---|
| HPBC | 0,50 | 1,50 | 2,50 |
| Distearyldimethylammoniumchlorid | 0,6 | 0,6 | 0,6 |
| Glycerinmonodistearat | 0,2 | 0,2 | 0,2 |
| Polyoxyethylen-(45)-polyoxypropylenè(33)-monobutylether | 0,15 | 0,15 | 0,15 |
| Stearylalkohol | 0,1 | 0,1 | 0,1 |
| Stearylbetain | 0,09 | 0,09 | 0,09 |
| p-Hydroxybenzoesäuremethylester | 0,1 | 0,1 | 0,1 |
| Wasser | ad 100 | ad 100 | ad 100 |

### Beispiel 17: Haarstylinggel

| | A | B | C | D |
|---|---|---|---|---|
| HPBC | 0,50 | 1,50 | 2,50 | 4,00 |
| Polyvinylpyrrolidon | 3 | 3 | 3 | 3 |
| Polyacrylsäure (Carbomer) | 0,5 | 0,5 | 0,5 | 0,5 |
| Aminomethylpropanol | 0,39 | 0,39 | 0,39 | 0,39 |
| Ethanol | 12 | 12 | 12 | 12 |
| Wasser | ad 100 | ad 100 | ad 100 | ad 100 |

### Beispiel 18: Haarspray

| | A | B | C |
|---|---|---|---|
| HPBC | 0,50 | 1,50 | 2,50 |
| Vinylpyrrolidon/Vinylacetat Copolymer | 2,5 | 2,5 | 2,5 |
| Acrylates/Acrylamide Copolymer ¹⁾ | 2,5 | 2,5 | 2,5 |
| Parfümöl | 0,2 | 0,2 | 0,2 |
| n-Butan | 60 | 60 | 60 |
| Ethanol | ad 100 | ad 100 | ad 100 |

| | | | |
|---|---|---|---|
| ¹⁾ Ultrahold® 8, neutralisiert mit Aminomethylpropanol | | | |

### Beispiel 19: Versprühbares Haarpflegemittel, nicht auszuspülen

| | A | B | C | D |
|---|---|---|---|---|
| HPBC | 0,50 | 1,50 | 2,50 | 4,00 |
| Polyvinylpyrrolidon | 0,9 | 0,9 | 0,9 | 0,9 |
| Cetyltrimethylammoniumchlorid | 0,4 | 0,4 | 0,4 | 0,4 |
| Cocamidopropylbetain | 0,33 | 0,33 | 0,33 | 0,33 |
| Zitronensäure | 0,2 | 0,2 | 0,2 | 0,2 |
| Isopropanol | 0,28 | 0,28 | 0,28 | 0,28 |
| Keratinhydrolysat | 0,1 | 0,1 | 0,1 | 0,1 |
| Wasser | ad 100 | ad 100 | ad 100 | ad 100 |

### Beispiel 20: Versprühbares Haarpflegemittel, nicht auszuspülen

| | A | B | C |
|---|---|---|---|
| Sphärische Mikropartikel | 0,50 | 1,50 | 2,50 |
| Polyvinylpyrrolidon | 0,8 | 0,8 | 0,8 |
| Vinylimidazoliniummethochlorid/Vinylpyrrolidon Copolymer | 0,32 | 0,32 | 0,32 |
| Kokosfettsäureamidopropylbetain | 0,21 | 0,21 | 0,21 |
| PEG-7-hydrogenated caster oil | 0,05 | 0,05 | 0,05 |
| PEG-40-hydrogenated caster oil | 0,18 | 0,18 | 0,18 |
| Keratinhydrolisat | 0,2 | 0,2 | 0,2 |
| Ethanol | 12 | 12 | 12 |
| Glyoxylsäure | 0,2 | 0,2 | 0,2 |
| Wasser | ad 100 | ad 100 | ad 100 |

### Beispiel 21: Dauerverformungsmittel

| | A | B | C | D |
|---|---|---|---|---|
| HPBC | 0,50 | 1,50 | 2,50 | 4,00 |
| Thioglykolsäure | 8,82 | 8,82 | 8,82 | 8,82 |
| PEG-35-caster oil | 0,90 | 0,90 | 0,90 | 0,90 |
| Polyethylenglykol-(23)-nonylphenolether | 0,90 | 0,90 | 0,90 | 0,90 |
| Poly(diallyldimethylammoniumchlorid) | 0,90 | 0,90 | 0,90 | 0,90 |
| Styrol/Polyvinylpyrrolidon Copolymer | 0,28 | 0,28 | 0,28 | 0,28 |
| Ammoniak | 0,15 | 0,15 | 0,15 | 0,15 |
| Ammoniumhydrogencarbonat | 3,5 | 3,5 | 3,5 | 3,5 |
| Parfümöl | 0,5 | 0,5 | 0,5 | 0,5 |
| Wasser | ad 100 | ad 100 | ad 100 | ad 100 |

### Beispiel 22: Fixiermittel für die Dauerverformung

| | A | B | C | D |
|---|---|---|---|---|
| HPBC | 0,50 | 1,50 | 2,50 | 4,00 |
| Cetylstearylalkohol | 2,61 | 2,61 | 2,61 | 2,61 |
| Natriumlaurylsulfat | 0,29 | 0,29 | 0,29 | 0,29 |
| Lanolinalkohol | 0,4 | 0,4 | 0,4 | 0,4 |
| Wasserstoffperoxid | 1,9 | 1,9 | 1,9 | 1,9 |
| Phosphorsäure | 0,085 | 0,085 | 0,085 | 0,085 |
| Salicylsäure | 0,1 | 0,1 | 0,1 | 0,1 |
| Parfümöl | 0,3 | 0,3 | 0,3 | 0,3 |
| Wasser | ad 100 | ad 100 | ad 100 | ad 100 |

### Beispiel 23: Schaumfixiermittel

| | A | B | C | D |
|---|---|---|---|---|
| Sphärische Mikropartikel | 0,50 | 1,50 | 2,50 | 4,00 |
| Wasserstoffperoxid (35%ig) | 14 | 14 | 14 | 14 |
| o-Phosphorsäure (85%ig) | 3,41 | 3,41 | 3,41 | 3,41 |
| DL-2-Pyrrolidon-5-carbonsäure | 1,25 | 1,25 | 1,25 | 1,25 |
| Laurylaminodimethylacetobetain | 0,66 | 0,66 | 0,66 | 0,66 |
| Polypropylen-(1)-polyethylen-(9)-laurylglykolether | 0,5 | 0,5 | 0,5 | 0,5 |
| Parfüm | 0,2 | 0,2 | 0,2 | 0,2 |
| p-Acetaminophenol | 0,05 | 0,05 | 0,05 | 0,05 |
| Wasser | ad 100 | ad 100 | ad 100 | ad 100 |

### Beispiel 24: Haartönungsschaum

| | A | B | C | D |
|---|---|---|---|---|
| HPBC | 0,50 | 1,50 | 2,50 | 4,00 |
| Cetylstearylalkohol | 4,5 | 4,5 | 4,5 | 4,5 |
| Cetyltrimethylammoniumchlorid | 1,75 | 1,75 | 1,75 | 1,75 |
| Isopropanol | 4,2 | 4,2 | 4,2 | 4,2 |
| Basic Violet 14 (C.I. 42510) | 0,0025 | 0,0025 | 0,0025 | 0,0025 |
| Wasser | ad 100 | ad 100 | ad 100 | ad 100 |

94,00 g des vorstehenden Haartönungsschaumes werden mit 6 g eines Gemisches aus 50 Gew.% Propan, 40 Gew.% n-Butan und 10 Gew.% Dimethylether in einen Aerosolbehälter abgefüllt. Das erfindungsgemässe Haartönungsmittel wird beim Versprühen dem Behälter in Schaumform entnommen.

### Beispiel 25: Oxidatives Haarfärbemittel (dunkelbraun)

| | A | B | C | D |
|---|---|---|---|---|
| HPBC | 0,50 | 1,50 | 2,50 | 4,00 |
| Cetylstearylalkohol | 17,1 | 17,1 | 17,1 | 17,1 |
| Natriumlaurylsulfat | 1,9 | 1,9 | 1,9 | 1,9 |
| Lanolinalkohol | 2,1 | 2,1 | 2,1 | 2,1 |
| Glycerylstearat | 6,1 | 6,1 | 6,1 | 6,1 |
| Natriumcocoylisoethionat | 0,4 | 0,4 | 0,4 | 0,4 |
| Natriumlaurylethersulfat | 1,4 | 1,4 | 1,4 | 1,4 |
| p-Aminophenolhydrochlorid | 0,06 | 0,06 | 0,06 | 0,06 |
| p-Toluylendiaminsulfat | 0,65 | 0,65 | 0,65 | 0,65 |
| Resorcin | 0,26 | 0,26 | 0,26 | 0,26 |
| Natriumsulfit | 0,6 | 0,6 | 0,6 | 0,6 |
| Ethylendiamintetraessigsäure | 0,3 | 0,3 | 0,3 | 0,3 |
| Isopropanol | 6 | 6 | 6 | 6 |
| Parfümöl | 0,3 | 0,3 | 0,3 | 0,3 |
| Ammoniak | 1,4 | 1,4 | 1,4 | 1,4 |
| Wasser | ad 100 | ad 100 | ad 100 | Ad 100 |

Vor der Anwendung werden 50 g des vorstehenden Mittels mit 50 ml Wasserstoffperoxid (6%ige Lösung) vermischt.

### Beispiel 26: Festigendes Aerosol-Gelée

| | A | B | C | D |
|---|---|---|---|---|
| HPBC | 2,00 | 3,00 | 3,50 | 4,00 |
| Polyvinylpyrrolidon | 5,00 | - | - | 3,00 |
| Vinylpyrrolidon/Vinylacetat Copolymer | - | 3,50 | - | - |
| Acrylates/Acrylamide Copolymer¹⁾ | | - | 3,00 | - |
| PEG-40-Hydrogenated Castor Oil | 0,15 | 0,20 | 0,15 | 0,25 |
| Ethanol | 40,00 | 40,00 | 45,00 | 40,00 |
| Ceteareth-12 | 0,15 | 0,20 | 0,2 | 0,25 |
| Parfum | 0,2 | 0,3 | 0,25 | 0,3 |
| Dimethylether | 35 | 35 | 30 | 30 |
| Wasser | ad 100 | ad 100 | ad 100 | ad 100 |

| | | | | |
|---|---|---|---|---|
| ¹⁾ Ultrahold® 8, neutralisiert mit Aminomethylpropanol | | | | |

Die Applikation erfolgt aus einer Glasaerosol- oder Aluminium-Aerosolverpackung mit einem geeigneten Sprühventil.

## Patentansprüche

1. Verwendung mindestens eines N-Hydroxyalkyl-O-benzylchitosans zur Haarbehandlung.

2. Verwendung nach Anspruch 1, **dadurch gekennzeichnet, dass** die Hydroxyalkylgruppe eine oder mehrere Hydroxygruppen und 2 bis 20 C-Atome aufweist.

3. Verwendung nach einem der vorhergehenden Ansprüche, **dadurch gekennzeichnet, dass** die Hydroxyalkylgruppe ausgewählt ist aus Hydroxyethyl, Hydroxypropyl und Hydroxybutyl.

4. Verwendung nach Anspruch 1, **dadurch gekennzeichnet, dass** das N-Hydroxyalkyl-O-benzylchitosan erhältlich ist aus Chitosan durch N-Hydrxyalkylierung mit einem Alkylenoxid und gleichzeitiger oder anschließender O-Benzylierung mit einer reaktiven Benzylverbindung.

5. Verwendung nach Anspruch 4, **dadurch gekennzeichnet, dass** das Alkylenoxid ausgewählt ist aus Ethylenoxid, Propylenoxid und Butylenoxid und/oder die Benzylverbindung ausgewählt ist aus Benzylhalogeniden.

6. Verwendung nach einem der vorhergehenden Ansprüche, **dadurch gekennzeichnet, dass** das N-Hydroxyalkyl-O-benzylchitosan mindestens eine Einheit der allgemeinen Formel 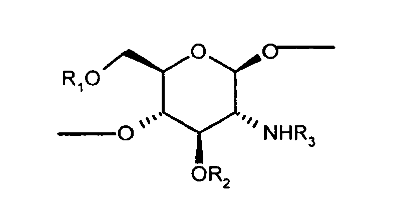 aufweist, wobei R¹ und R² unabhängig voneinander eine Gruppe -(A-O)ₙ-H bedeuten, A eine C2- bis C20-Alkylengruppe ist, n eine Zahl größer gleich Null ist und R³ für Wasserstoff, -CH₂Ph oder -CO-CH₃ steht,
mit der Maßgabe, dass bei mindestens einer Einheit n größer Null und R³ gleich -CH₂Ph sind und/oder dass bei mindestens einer ersten Einheit n größer Null und R³ gleich Wasserstoff oder -CO-CH₃ und bei mindestens einer zweiten Einheit n gleich Null und R³ gleich -CH₂Ph sind.

7. Haarbehandlungsmittel mit einem Gehalt an
(A) mindestens einem N-Hydroxyalkyl-O-benzylchitosan gemäß der Definition nach einem der Ansprüche 1 bis 6 und
(B) mindestens einem weiteren Haarbehandlungswirkstoff, ausgewählt aus haarpflegenden, haarschützenden, haarreparierenden, haarreinigenden, haarfärbenden, haarfestigenden und die Haarform verändernden Wirkstoffen, wobei die haarfärbenden Wirkstoffe ausgwählt sind aus Oxidationshaarfarbstoffvorprodukten, direktiehenden Haarfarbstoffen und haarfärbenden Pigmenten, wobei die haarfärbenden Pigmente ausgewählt sind aus schwarzem Eisenoxid (CI 77499), gelbem Eisenoxid (CI 77492), Manganviolett (CI 77742), Ultramarine (CI 77007), Chromoxidhydrat (CI77289), Eisenblau (CI77510), Carmine (Cochineal), Perlglanz- und Farbpigmenten auf Micabasis welche mit einem Metalloxid oder einem Metalloxychlorid wie Titandioxid oder Wismutoxychlorid sowie gegebenenfalls weiteren farbgebenden Stoffen wie Eisenoxiden, Eisenblau, Ultramarine oder Carmine beschichtet sind und wobei die Farbe durch Variation der Schichtdicke bestimmt ist.

8. Mittel nach Anspruch 7, **dadurch gekennzeichnet, dass** der Wirkstoff (B) ausgewählt ist aus haarpflegenden Tensiden, haareinigenden Tensiden, haarpflegenden Ölen und Wachsen, haarpflegenden Polymeren, haarpflegenden Silikonverbindungen, haarfestigenden Polymeren, Lichtschutzstoffen, Oxidationsmitteln und Keratin reduzierenden Stoffen.

9. Mittel nach einem der Ansprüche 7 bis 8, **dadurch gekennzeichnet, dass** das N-Hydroxyalkyl-O-benzylchitosan (A) in einer Menge von 0,01 bis 20 Gew.% und der Wirkstoff (B) in einer Menge von 0,01 bis 20 Gew.% enthalten ist.

10. Mittel nach einem der Ansprüche 7 bis 9, **dadurch gekennzeichnet, dass** als haarpflegendes oder haarfestigendes Polymer ein Polymer mit anionischen oder anionisierbaren Gruppen enthalten ist, ausgewählt aus Terpolymeren aus Acrylsäure, Ethylacrylat und N-tert-Butylacrylamid; vernetzten oder unvernetzten Vinylacetat/Crotonsäure Copolymeren; Terpolymeren aus tert.-Butylacrylat, Ethylacrylat und Methacrylsäure; Natriumpolystyrolsulfonat; Copolymeren aus Vinylacetat, Crotonsäure und Vinylpropionat; Copolymeren aus Vinylacetat, Crotonsäure und Vinylneodecanoat; Aminomethylpropanol-Acrylat Copolymeren; Copolymeren aus Vinylpyrrolidon und mindestens einem weiteren Monomer ausgewählt aus Acrylsäure, Methacrylsäure, Acrylsäureestern und Methacrylsäureestern; Copolymeren aus Methylvinylether und Maleinsäuremonoalkylestern; Aminomethylpropanolsalze von Copolymeren aus Allylmethacrylat und mindestens einem weiteren Monomer ausgewählt aus Acrylsäure, Methacrylsäure, Acrylsäureestern und Methacrylsäureestern; vernetzten Copolymeren aus Ethylacrylat und Methacrylsäure; Copolymeren aus Vinylacetat, Mono-n-butylmaleat und Isobornylacrylat; Copolymeren aus zwei oder mehr Monomeren ausgewählt aus Acrylsäure, Methacrylsäure, Acrylsäureestern und Methacrylsäureestern, Copolymeren aus Octylacrylamid und mindestens einem Monomeren ausgewählt aus Acrylsäure, Methacrylsäure, Acrylsäureestern und Methacrylsäureestern; Polyestern aus Diglycol, Cyclohexandimethanol, Isophtalsäure und Sulfoisophtalsäure.

11. Mittel nach einem der Ansprüche 7 bis 10, **dadurch gekennzeichnet, dass** als haarpflegendes oder haarfestigendes Polymer ein Polymer mit kationischen oder kationisierbaren Gruppen enthalten ist, ausgewählt aus kationischen Cellulosederivaten aus Hydroxyethylcellulose und Diallyldimethylammoniumchlorid; kationischen Cellulosederivaten aus Hydroxyethylcellulose und mit Trimethylammonium substituiertem Epoxid; Poly(dimethyldiallylammoniumchlorid); Copolymeren aus Acrylamid und Dimethyldiallylammoniumchlorid; quaternären Ammoniumpolymeren, gebildet durch die Reaktion von Diethylsulfat und einem Copolymer aus Vinylpyrrolidon und Dimethylaminoethylmethacrylat; quaternären Ammoniumpolymeren aus Methylvinylimidazoliumchlorid und Vinylpyrrolidon; Polyquaternium-35; Polymer aus Trimethylammonium-ethyl-methacrylatchlorid; Polyquaternium-57; endständig mit quaternären Ammoniumgruppen substituierte Dimethylpolysiloxane; Copolymer aus Vinylpyrrolidon, Dimethylaminopropylmethacrylamid und Methacryloylaminopropyllauryldimethylammoniumchlorid; Chitosan und dessen Salze; Hydroxyalkylchitosane und deren Salze; Alkyl-hydroxyalkylchitosane und deren Salze; N-Hydroxyalkylchitosanalkylether; Copolymer aus Vinylcaprolactam, Vinylpyrrolidon und Dimethylaminoethylmethacrylat; Copolymeren aus Vinylpyrrolidon und Dimethylaminoethylmethacrylat, Copolymeren aus Vinylpyrrolidon, Vinylcaprolactam und Dimethylaminopropylacrylamid; Poly- oder Oligoestern, aufgebaut aus mindestens einer ersten Monomerart, die ausgewählt ist aus mit mindestens einer quaternären Ammoniumgruppe substituierten Hydroxysäure.

12. Mittel nach einem der Ansprüche 7 bis 11, **dadurch gekennzeichnet, dass** als haarpflegendes oder haarfestigendes Polymer ein zwitterionisches und/oder ein amphoteres Polymer enthalten ist, ausgewählt aus Copolymeren aus Octylacrylamid, Acrylsäure, Butylaminoethylmethacrylat, Methylmethacrylat und Hydroxypropylmethacrylat; Copolymeren aus Laurylacrylat, Stearylacrylat, Ethylaminoxidmethacrylat und mindestens einem Monomeren ausgewählt aus Acrylsäure, Methacrylsäure, Acrylsäureestern und Methacrylsäureestern; Copolymeren aus Methacryloylethylbetain und mindestens einem Monomeren ausgewählt aus Methacrylsäure und Methacrylsäureestern; Copolymeren aus Acrylsäure, Methylacrylat und Methacrylamidopropyltrimethylammoniumchlorid; Oligomeren oder Polymeren, herstellbar aus quaternären Crotonbetainen oder quaternären Crotonbetainestern.

13. Mittel nach einem der Ansprüche 7 bis 12, **dadurch gekennzeichnet, dass** als haarpflegendes oder haarfestigendes Polymer ein nichtionisches Polymer enthalten ist, ausgewählt aus Polyvinylpyrrolidon, Polyvinylcaprolactam, Vinylpyrrolidon/Vinylacetat Copolymeren, Polyvinylalkohol, Isobutylen/Ethylmaleimid/Hydroxyethylmaleimid Copolymer; Copolymyeren aus Vinylpyrrolidon, Vinylacetat und Vinylpropionat.

14. Mittel nach einem der Ansprüche 7 bis 13, **dadurch gekennzeichnet, dass** als haarpflegende Silikonverbindung mindestens eine Silikonverbindung enthalten ist, ausgewählt aus cyclischen Dimethylsiloxanen, linearen Polydimethylsiloxanen, Blockpolymeren aus Polydimethylsiloxan und Polyethylenoxid und/oder Polypropylenoxid, Polydimethylsiloxanen mit end- oder seitenständigen Polyethylenoxid- oder Polypropylenoxidresten, Polydimethylsiloxanen mit endständigen Hydroxylgruppen, phenylsubstituierten Polydimethylsiloxanen, Silikonemulsionen, Silkonelastomeren, Silikonwachsen, Silikongums, aminosubstituierten Silikonen und mit einer oder mehreren quaternären Ammoniumgruppen substituierten Silikonen.

15. Mittel nach einem der vorhergehenden Mittelansprüche, **dadurch gekennzeichnet, dass** der Lichtschutzstoff ausgewählt ist aus 4-Methoxy-zimtsäure-2-ethylhexylester, Methylmethoxycinnamat, 2-Hydroxy-4-methoxybenzophenon-5-sulfonsäure und polyethoxylierten p-Aminobenzoaten.

16. Mittel nach einem der vorhergehenden Mittelansprüche, **dadurch gekennzeichnet, dass** als haarpflegendes Öl oder als haarpflegendes oder harfestigendes Wachs ein Öl oder Wachs enthalten ist, ausgewählt aus Paraffinwachsen, Polyolefinwachsen, Wollwachs, Wollwachsalkoholen, Candelillawachs, Olivenwachs, Carnaubawachs, Japanwachs, Apfelwachs, gehärteten Fetten, Fettsäureestern, Fettsäureglyceriden, Fettsäuretriglyceriden, Polyethylenglykolwachsen, Silikonwachsen, Silikonölen, Mineralölen, Isoparaffinölen, Paraffinölen, Squalan, Sonnenblumenöl, Kokosöl, Rizinusöl, Lanolinöl, Jojobaöl, Maisöl, Sojaöl.

17. Mittel nach einem der vorhergehenden Mittelansprüche, **dadurch gekennzeichnet, dass** der haarpflegende, haarschützende oder haarreparierende Wirkstoff (B) ausgewählt ist aus Betain; Panthenol; Panthenylethylether; Sorbitol; Proteinhydrolysaten; Pflanzenextrakten; A-B-Block-Copolymeren aus Alkylacrylaten und Alkylmethacrylaten; A-B-Block-Copolymeren aus Alkylmethacrylaten und Acrylnitril; A-B-A-Block-Copolymeren aus Lactid und Ethylenoxid; A-B-A-Block-Copolymeren aus Caprolacton und Ethylenoxid; A-B-C-Block-Copolymeren aus Alkylen- oder Alkadienverbindungen, Styrol und Alkylmethacrylaten; A-B-C-Block-Copolymeren aus Acrylsäure, Styrol und Alkylmethacrylaten; sternförmigen Block-Copolymeren; hyperverzweigten Polymeren, Dendrimeren, intrinsisch elektrisch leitfähigen 3,4-Polyethylendioxythiophenen und intrinsisch elektrisch leitfähigen Polyanilinen.

18. Mittel nach einem der vorhergehenden Mittelansprüche, **dadurch gekennzeichnet, dass** mindestens ein haarpflegendes Tensid enthalten ist, ausgewählt aus Anlagerungsprodukte von 2 bis 30 mol Ethylenoxid und/oder 1 bis 5 mol Propylenoxid an C8- bis C22-Fettalkohole, Anlagerungsprodukte von 2 bis 30 mol Ethylenoxid und/oder 1 bis 5 mol Propylenoxid an C12- bis C22-Fettsäuren, Anlagerungsprodukte von 2 bis 30 mol Ethylenoxid und/oder 1 bis 5 mol Propylenoxid an Alkylphenole mit 8 bis 15 C-Atomen in der Alkylgruppe, C12- bis C22-Fettsäuremono- und -diester von Anlagerungsprodukten von 1 bis 30 mol Ethylenoxid an Glycerin, Anlagerungsprodukte von 5 bis 60 mol Ethylenoxid an Rizinusöl oder an gehärtetes (hydriertes) Rizinusöl, Mono-, Di- oder Triester der Phosphorsäure mit Anlagerungsprodukten von 2 bis 30 mol Ethylenoxid an C8- bis C22-Fettalkohole, Ester aus Saccharose und ein oder zwei C8- bis C22-Fettsäuren, Ester aus Sorbitan und ein, zwei oder drei C8- bis C22-Fettsäuren und einem Ethoxylierungsgrad von 4 bis 20, Polyglycerylfettsäureester aus ein, zwei oder mehreren C8- bis C22-Fettsäuren und Polyglycerin mit 2 bis 20 Glyceryleinheiten, Alkylglykoside, C8-22-Alkyl-dimethylbenzylammoniumverbindungen, C8-22-Alkyltrimethylammoniumverbindungen, C8-22-Alkyldimethylhydroxyethylammoniumverbindungen, Di-(C8-22-alkyl)-dimethylammoniumverbindungen, C8-22-Alkylpyridiniumsalze, C8-22-Alkylamidoethyltrimethylammoniumethersulfate, C8-22-Alkylmethylaminoxide, C8-22-Alkylaminoethyldimethylaminoxide, Amidoaminen und quaternisierten Amidoaminen.

19. Mittel nach einem der Ansprüche 7 bis 18, **dadurch gekennzeichnet, dass** es in Form eines Haargels, in Form einer viskosen Haarlotion oder in Form eines Haarsprühgels, welches mit einer mechanischen Vorrichtung versprüht wird, vorliegt, zusätzlich mindestens ein verdickendes Polymer enthält und eine Viskosität von mindestens 250 mPa s (gemessen mit einem Bohlin Rheometer CS, Messkörper C25 bei 25°C und einer Schergeschwindigkeit von 50 s⁻¹) aufweist.

20. Mittel nach einem der Ansprüche 7 bis 18, **dadurch gekennzeichnet, dass** es in Form einer O/W-Emulsion, einer W/O-Emulsion oder einer Mikroemulsion vorliegt und mindestens ein Öl oder Wachs sowie mindestens einen Emulgator enthält.

21. Mittel nach einem der Ansprüche 7 bis 18 in Form eines Haarsprays, **dadurch gekennzeichnet, dass** es entweder in Kombination mit einer mechanischen Pumpsprühvorrichtung oder in Kombination mit mindestens einem Treibmittel, ausgewählt aus Propan, Butan, Dimethylether und fluorierten Kohlenwasserstoffen vorliegt.

22. Mittel nach einem der Ansprüche 7 bis 18, **dadurch gekennzeichnet, dass** es in Form eines verschäumbaren Produktes in Kombination mit einer Vorrichtung zum Verschäumen vorliegt, mindestens ein schaumbildendes Tensid oder mindestens ein schaumbildendes Polymer enthält und entweder in Kombination mit einer mechanischen Pumpschäumvorrichtung oder in Kombination mit mindestens einem Treibmittel, ausgewählt aus Propan, Butan, Dimethylether und fluorierten Kohlenwasserstoffen vorliegt.

23. Mittel nach einem der Ansprüche 7 bis 18, **dadurch gekennzeichnet, dass** es in Form eines festen Haarwachses vorliegt und mindestens eines der in Anspruch 17 genannten Wachse enthält.

24. Mittel nach einem der Ansprüche 7 bis 18, **dadurch gekennzeichnet, dass** es in Form eines Haareinigungsmittels vorliegt und 0,01 bis 40 Gew.% mindestens eines anionischen, amphoteren oder nicht-ionischen haarreinigenden Tensids und 50 bis 90 Gew.% Wasser enthält.

25. Mittel nach einem der Ansprüche 7 bis 18, **dadurch gekennzeichnet, dass** es ein Dauerverformungsmittel ist und mindestens einen Keratin reduzierenden Stoff enthält, ausgewählt aus keratinreduzierenden Mercaptoverbindungen.

26. Mittel nach einem der Ansprüche 7 bis 18, **dadurch gekennzeichnet, dass** es ein Haarblondiermittel, ein Oxidationshaarfärbemittel oder ein Dauerwellfixiermittel ist mit einem Gehalt an mindestens einem Oxidationsmittel.

27. Mittel nach einem der Ansprüche 7 bis 18, **dadurch gekennzeichnet, dass** es ein Haartönungsmittel oder ein Oxidationshaarfärbemittel ist und mindestens einen direkt auf das Haar aufziehenden synthetischen und/oder natürlichen Haarfarbstoff oder mindestens eine Oxidationsfarbstoffvorstufe enthält.

28. Verfahren zur Haarbehandlung, wobei
- ein Haarbehandlungsmittel nach einem der Ansprüche 7 bis 27 bereit gestellt wird,
- das Haarbehandlungsmittel auf das Haar aufgebracht wird und
- das Haarbehandlungsmittel entweder nach einer Einwirkzeit aus dem Haar ausgepült oder im Haar belassen wird.

## Claims

1. Use of at least one N-hydroxyalkyl-O-benzylchitosan in hair treatment.

2. Use according to Claim 1, **characterized in that** the hydroxyalkyl group has one or more hydroxy groups and 2 to 20 carbon atoms.

3. Use according to one of the preceding claims, **characterized in that** the hydroxyalkyl group is selected from hydroxyethyl, hydroxypropyl and hydroxybutyl.

4. Use according to Claim 1, **characterized in that** the N-hydroxyalkyl-O-benzylchitosan is obtainable from chitosan by N-hydroxyalkylation with an alkylene oxide and simultaneous or subsequent O-benzylation with a reactive benzyl compound.

5. Use according to Claim 4, **characterized in that** the alkylene oxide is selected from ethylene oxide, propylene oxide and butylene oxide and/or the benzyl compound is selected from benzyl halides.

6. Use according to one of the preceding claims, **characterized in that** the N-hydroxyalkyl-O-benzylchitosan has at least one unit of the general formula 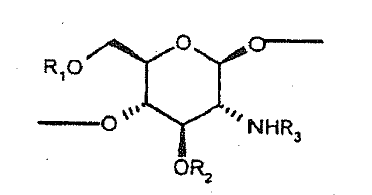 where R¹ and R², independently of one another, are a group -(A-O)ₙ-H, A is a C2- to C20-alkylene group, n is a number greater than or equal to zero and R³ is hydrogen, -CH₂Ph or -CO-CH₃,
with the proviso that in the case of at least one unit n is greater than zero and R³ is -CH₂Ph and/or that in the case of at least one first unit n is greater than zero and R³ is hydrogen or -CO-CH₃ and in the case of at least one second unit n is zero and R³ is -CH₂Ph.

7. Hair-treatment agent with a content of
(A) at least one N-hydroxyalkyl-O-benzylchitosan according to the definition according to one of Claims 1 to 6 and
(B) at least one further hair-treatment active ingredient selected from haircare, hair-protecting, hair-repairing, hair-cleansing, hair-colouring, hair-setting and hairstyling active ingredients, where the hair-colouring active ingredients are selected from oxidation hair dye precursors, direct hair dyes and hair-colouring pigments, where the hair-colouring pigments are selected from black iron oxide (CI 77499), yellow iron oxide (CI 77492), manganese violet (CI 77742), ultramarine (CI 77007), chromium oxide hydrate (CI 77289), iron blue (CI 77510), carmine (cochineal), pearlescent pigments and coloured pigments based on mica which are coated with a metal oxide or a metal oxychloride, such as titanium dioxide or bismuth oxychloride, and optionally further colour-impairing substances, such as iron oxides, iron blue, ultramarine or carmine, and where the colour is determined by varying the layer thickness.

8. Agent according to Claim 7, **characterized in that** the active ingredient (B) is selected from haircare surfactants, hair-cleaning surfactants, haircare oils and waxes, haircare polymers, haircare silicone compounds, hair-setting polymers, photoprotective substances, oxidizing agents and keratin-reducing substances.

9. Agent according to one of Claims 7 to 8, **characterized in that** the N-hydroxyalkyl-O-benzylchitosan (A) is present in an amount of from 0.01 to 20% by weight, and the active ingredient (B) is present in an amount of from 0.01 to 20% by weight.

10. Agent according to one of Claims 7 to 9, **characterized in that** the haircare or hair-setting polymer present is a polymer with anionic or anionizable groups selected from terpolymers of acrylic acid, ethyl acrylate and N-tert-butylacrylamide; crosslinked or uncrosslinked vinyl acetate/crotonic acid copolymers; terpolymers of tert-butyl acrylate, ethyl acrylate and methacrylic acid; sodium polystyrenesulphonate; copolymers of vinyl acetate, crotonic acid and vinyl propionate; copolymers of vinyl acetate, crotonic acid and vinyl neodecanoate; aminomethylpropanol-acrylate copolymers; copolymers of vinylpyrrolidone and at least one further monomer selected from acrylic acid, methacrylic acid, acrylic acid esters and methacrylic acid esters; copolymers of methyl vinyl ether and maleic acid monoalkyl esters; aminomethylpropanol salts of copolymers of allyl methacrylate and at least one further monomer selected from acrylic acid, methacrylic acid, acrylic acid esters and methacrylic acid esters; crosslinked copolymers of ethyl acrylate and methacrylic acid; copolymers of vinyl acetate, mono-n-butyl maleate and isobornyl acrylate; copolymers of two or more monomers selected from acrylic acid, methacrylic acid, acrylic acid esters and methacrylic acid esters, copolymers of octylacrylamide and at least one monomer selected from acrylic acid, methacrylic acid, acrylic acid esters and methacrylic acid esters; polyesters of diglycol, cyclohexanedimethanol, isophthalic acid and sulphoisophthalic acid.

11. Agent according to one of Claims 7 to 10, **characterized in that** the haircare or hair-setting polymer present is a polymer with cationic or cationizable groups selected from cationic cellulose derivatives of hydroxyethylcellulose and diallyldimethylammonium chloride; cationic cellulose derivatives of hydroxyethylcellulose and epoxide substituted with trimethylammonium; poly(dimethyldiallylammonium chloride); copolymers of acrylamide and dimethyldiallylammonium chloride; quaternary ammonium polymers formed by the reaction of diethyl sulphate and a copolymer of vinylpyrrolidone and dimethylaminoethyl methacrylate; quaternary ammonium polymers of methylvinylimidazolium chloride and vinylpyrrolidone; polyquaternium-35; polymer of trimethylammonium-ethyl methacrylate chloride; polyquaternium-57; dimethylpolysiloxanes terminally substituted by quaternary ammonium groups; copolymer of vinylpyrrolidone, dimethylaminopropylmethacrylamide and methacryloylaminopropyllauryldimethylammonium chloride; chitosan and salts thereof; hydroxyalkylchitosans and salts thereof; alkylhydroxyalkylchitosans and salts thereof; N-hydroxyalkylchitosan alkyl ethers; copolymer of vinylcaprolactam, vinylpyrrolidone and dimethylaminoethyl methacrylate; copolymers of vinylpyrrolidone and dimethylaminoethyl methacrylate, copolymers of vinylpyrrolidone, vinylcaprolactam and dimethylaminopropylacrylamide; polyesters or oligoesters composed of at least one first type of monomer which is selected from hydroxy acid substituted by at least one quaternary ammonium group.

12. Agent according to one of Claims 7 to 11, **characterized in that** the haircare or hair-setting polymer present is a zwitterionic and/or an amphoteric polymer selected from copolymers of octylacrylamide, acrylic acid, butylaminoethyl methacrylate, methyl methacrylate and hydroxypropyl methacrylate; copolymers of lauryl acrylate, stearyl acrylate, ethylamine oxide methacrylate and at least one monomer selected from acrylic acid, methacrylic acid, acrylic acid esters and methacrylic acid esters; copolymers of methacryloylethylbetaine and at least one monomer selected from methacrylic acid and methacrylic acid esters; copolymers of acrylic acid, methyl acrylate and methacrylamidopropyltrimethylammonium chloride; oligomers or polymers producible from quaternary croton betaines or quaternary croton betaine esters.

13. Agent according to one of Claims 7 to 12, **characterized in that** the haircare or hair-setting polymer present is a nonionic polymer selected from polyvinylpyrrolidone, polyvinylcaprolactam, vinylpyrrolidone/vinyl acetate copolymers, polyvinyl alcohol, isobutylene/ethyl maleimide/hydroxyethylmaleimide copolymer; copolymers of vinylpyrrolidone, vinyl acetate and vinyl propionate.

14. Agent according to one of Claims 7 to 13, **characterized in that** the haircare silicone compound present is at least one silicone compound selected from cyclic dimethylsiloxanes, linear polydimethylsiloxanes, block polymers of polydimethylsiloxane and polyethylene oxide and/or polypropylene oxide, polydimethylsiloxanes with terminal or lateral polyethylene oxide or polypropylene oxide radicals, polydimethylsiloxanes with terminal hydroxyl groups, phenyl-substituted polydimethylsiloxanes, silicone emulsions, silicone elastomers, silicone waxes, silicone gums, amino-substituted silicones and silicones substituted by one or more quaternary ammonium groups.

15. Agent according to one of the preceding agent claims, **characterized in that** the photoprotective substance is selected from 2-ethylhexyl 4-methoxycinnamate, methyl methoxycinnamate, 2-hydroxy-4-methoxybenzophenone-5-sulphonic acid and polyethoxylated p-aminobenzoates.

16. Agent according to one of the preceding agent claims, **characterized in that** the haircare oil or haircare or hair-setting wax present is an oil or wax selected from paraffin waxes, polyolefin waxes, wool wax, wool wax alcohols, candelilla wax, olive wax, carnauba wax, japan wax, apple wax, hydrogenated fats, fatty acid esters, fatty acid glycerides, fatty acid triglycerides, polyethylene glycol waxes, silicone waxes, silicone oils, mineral oils, isoparaffin oils, paraffin oils, squalane, sunflower oil, coconut oil, castor oil, lanolin oil, jojoba oil, corn oil, soya oil.

17. Agent according to one of the preceding agent claims, **characterized in that** the haircare, hair-protecting or hair-repairing active ingredient (B) is selected from betaine; panthenol; panthenyl ethyl ether; sorbitol; protein hydrolysates; plant extracts; A-B block copolymers of alkyl acrylates and alkyl methacrylates; A-B block copolymers of alkyl methacrylates and acrylonitrile; A-B-A block copolymers of lactide and ethylene oxide; A-B-A block copolymers of caprolactone and ethylene oxide; A-B-C block copolymers of alkylene or alkadiene compounds, styrene and alkyl methacrylates; A-B-C block copolymers of acrylic acid, styrene and alkyl methacrylates; star-shaped block copolymers; hyperbranched polymers, dendrimers, intrinsically electrically conductive 3,4-polyethylene dioxythiophenes and intrinsically electrically conductive polyanilines.

18. Agent according to one of the preceding agent claims, **characterized in that** at least one haircare surfactant is present selected from addition products of from 2 to 30 mol of ethylene oxide and/or 1 to 5 mol of propylene oxide onto C8- to C22-fatty alcohols, addition products of from 2 to 30 mol of ethylene oxide and/or 1 to 5 mol of propylene oxide onto C12- to C22-fatty acids, addition products of from 2 to 30 mol of ethylene oxide and/or 1 to 5 mol of propylene oxide onto alkylphenols having 8 to 15 carbon atoms in the alkyl group, C12- to C22-fatty acid mono- and diesters of addition products of from 1 to 30 mol of ethylene oxide onto glycerol, addition products of from 5 to 60 mol of ethylene oxide onto castor oil or onto hydrogenated (hydrated) castor oil, mono-, di- or triesters of phosphoric acid with addition products of from 2 to 30 mol of ethylene oxide onto C8- to C22-fatty alcohols, esters of sucrose and one or two C8- to C22-fatty acids, esters of sorbitan and one, two or three C8- to C22-fatty acids and a degree of ethoxylation from 4 to 20, polyglyceryl fatty acid esters of one, two or more C8- to C22-fatty acids and polyglycerols having 2 to 20 glyceryl units, alkyl glycosides, C8-22-alkyldimethylbenzylammonium compounds, C8-22-alkyltrimethylammonium compounds, C8-22-alkyldimethylhydroxyethylammonium compounds, di(C8-22-alkyl)dimethylammonium compounds, C8-22-alkylpyridinium salts, C8-22-alkylamidoethyltrimethylammonium ether sulphates, C8-22-alkylmethylamine oxides, C8-22-alkylaminoethyldimethylamine oxides, amidoamines and quaternized amidoamines.

19. Agent according to one of Claims 7 to 18, **characterized in that** it is present in the form of a hair gel, in the form of a viscous hair lotion or in the form of a hairspray which is sprayed using a mechanical device, additionally comprises at least one thickening polymer and has a viscosity of at least 250 mPa s (measured using a Bohlin rheometer CS, measurement body C25 at 25°C and a shear rate of 50 s⁻¹).

20. Agent according to one of Claims 7 to 18, **characterized in that** it is in the form of an O/W emulsion, a W/O emulsion or a microemulsion and comprises at least one oil or wax and at least one emulsifier.

21. Agent according to one of Claims 7 to 18 in the form of a hairspray, **characterized in that** it is present either in combination with a mechanical pump spray device or in combination with at least one propellant selected from propane, butane, dimethyl ether and fluorinated hydrocarbons.

22. Agent according to one of Claims 7 to 18, **characterized in that** it is in the form of a foamable product in combination with a foaming device, comprises at least one foam-forming surfactant and at least one foam-forming polymer and is present either in combination with a mechanical pump foaming device or in combination with at least one propellant selected from propane, butane, dimethyl ether and fluorinated hydrocarbons.

23. Agent according to one of Claims 7 to 18, **characterized in that** it is in the form of a solid hair wax and comprises at least one of the waxes specified in Claim 17.

24. Agent according to one of Claims 7 to 18, **characterized in that** it is in the form of a hair-cleansing agent and comprises 0.01 to 40% by weight of at least one anionic, amphoteric or nonionic hair-cleansing surfactant and 50 to 90% by weight of water.

25. Agent according to one of Claims 7 to 18, **characterized in that** it is a permanent shaping agent and comprises at least one keratin-reducing substance selected from keratin-reducing mercapto compounds.

26. Agent according to one of Claims 7 to 18, **characterized in that** it is a hair bleaching agent, an oxidation hair colorant or a permanent-waving neutralizer with a content of at least one oxidizing agent.

27. Agent according to one of Claims 7 to 18, **characterized in that** it is a hair-tinting agent or an oxidation hair colorant and comprises at least one synthetic and/or natural hair dye that absorbs directly on the hair or at least one oxidation dye precursor.

28. Method of treating hair, where
- a hair-treatment agent according to one of Claims 7 to 27 is prepared,
- the hair-treatment agent is applied to the hair and
- the hair-treatment agent is either rinsed out of the hair after a contact time, or is left in the hair.

## Revendications

1. Utilisation d'au moins un N-hydroxyalkyl-O-benzylchitosane pour le traitement capillaire.

2. Utilisation selon la revendication 1, **caractérisée en ce que** le groupe hydroxyalkyle comporte un ou plusieurs groupes hydroxy et de 2 à 20 atomes de carbone.

3. Utilisation selon l'une quelconque des revendications précédentes, **caractérisée en ce que** le groupe hydroxyalkyle est choisi parmi les groupes hydroxyéthyle, hydroxypropyle et hydroxybutyle.

4. Utilisation selon la revendication 1, **caractérisée en ce que** le N-hydroxyalkyl-O-benzylchitosane peut être obtenu à partir du chitosane par N-hydroxyalkylation avec un oxyde d'alkylène et O-benzylation simultanée ou subséquente avec un composé benzylique réactif.

5. Utilisation selon la revendication 4, **caractérisée en ce que** l'oxyde d'alkylène est choisi parmi l'oxyde d'éthylène, l'oxyde de propylène et l'oxyde de butylène et/ou le composé benzylique est choisi parmi les halogénures de benzyle.

6. Utilisation selon l'une quelconque des revendications précédentes, **caractérisée en ce que** le N-hydroxyalkyl-O-benzylchitosane comporte au moins une unité de formule générale 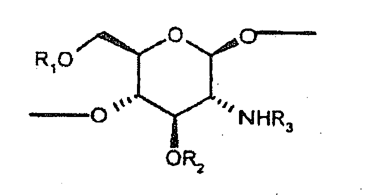 dans laquelle R¹ et R² représentent, indépendamment l'un de l'autre, un groupe -(A-O)ₙ-H, A est un groupe alkylène en C₂-C₂₀, n est une nombre supérieur ou égal à zéro et R³ représente un atome d'hydrogène, -CH₂Ph ou -CO-CH₃,
étant entendu que dans au moins une unité n est supérieur à zéro et R³ représente -CH₂Ph- et/ou **en ce que** dans au moins une première unité n est supérieur à zéro et R³ représente un atome d'hydrogène ou -CO-CH₃ et dans au moins une seconde unité n est égal à zéro et R³ représente -CH₂Ph.

7. Composition de traitement capillaire ayant une teneur en
(A) au moins un N-hydroxyalkyl-O-benzylchitosane conforme à la définition selon l'une quelconque des revendications 1 à 6 et
(B) au moins une autre substance active de traitement capillaire, choisie parmi des substances actives de soin capillaire, protégeant le cheveu, réparant le cheveu, nettoyant les cheveux, colorant les cheveux, fixant les cheveux et modifiant la forme des cheveux, les substances actives colorant les cheveux étant choisies parmi des précurseurs de colorants d'oxydation pour cheveux, des colorants directs pour cheveux et des pigments colorant les cheveux, les pigments colorant les cheveux étant choisis parmi l'oxyde de fer noir (CI 77499), l'oxyde de fer jaune (CI 77492), le violet de manganèse (CI 77742), l'outremer (CI 77007), l'hydrate de chrome (CI 77289), le bleu de Prusse (CI77510), les carmins (cochinéal), des pigments colorés et nacrés à base de mica qui sont enrobés d'un oxyde métallique ou d'un oxychlorure métallique tels que le dioxyde de titane ou l'oxychlorure de bismuth, ainsi qu'éventuellement d'autres substances colorantes telles que les oxydes de fer, le bleu de Prusse, les outremers ou les carmins et la couleur étant déterminée par la variation de l'épaisseur de couche.

8. Composition selon la revendication 7, **caractérisée en ce que** la substance active (B) est choisie parmi des tensioactifs de soin capillaire, des tensioactifs nettoyant lés cheveux, des huiles et cires de soin capillaire, des polymères de soin capillaire, des composés silicone de soin capillaire, des polymères fixant les cheveux, des substances photoprotectrices, des oxydants et des substances réduisant la kératine.

9. Composition selon la revendication 7 ou 8, **caractérisée en ce que** le N-hydroxyalkyl-O-benzylchitosane (A) est contenu en une quantité de 0,01 à 20 % en poids et la substance active (B) est contenue en une quantité de 0,01 à 20 % en poids.

10. Composition selon l'une quelconque des revendications 7 à 9, **caractérisée en ce qu'**en tant que polymère de soin capillaire ou fixant les cheveux est contenu un polymère à groupes anioniques ou pouvant être rendus anioniques, choisi parmi des terpolymères d'acide acrylique, acrylate d'éthyle et N-tert-butylacrylamide ; des copolymères acétate de vinyle/acide crotonique réticulés ou non réticulés ; des terpolymères d'acrylate de tert-butyle, acrylate d'éthyle et acide méthacrylique ; le polystyrènesulfonate de sodium ; des copolymères d'acétate de vinyle, acide crotonique et propionate de vinyle ; des copolymères d'acétate de vinyle, acide crotonique et néodécanoate de vinyle ; des copolymères aminométhylpropanol/acrylate ; des copolymères de vinylpyrrolidone et d'au moins un autre monomère choisi parmi l'acide acrylique, l'acide méthacrylique, des esters d'acide acrylique et des esters d'acide méthacrylique ; des copolymères d'oxyde de méthyle et de vinyle et de maléates de monoalkyle ; des sels avec l'aminométhylpropanol de copolymères de méthacrylate d'allyle et d'au moins un autre monomère choisi parmi l'acide acrylique, l'acide méthacrylique, des esters d'acide acrylique et des esters d'acide méthacrylique ; des copolymères réticulés d'acrylate d'éthyle et d'acide méthacrylique ; des copolymères d'acétate de vinyle, maléate de mono-n-butyle et acrylate d'isobornyle ; des copolymères de deux ou plus de deux monomères choisis parmi et d'au moins un autre monomère choisi parmi l'acide acrylique, l'acide méthacrylique, des esters d'acide acrylique et des esters d'acide méthacrylique ; des copolymères d'octylacrylamide et d'au moins un monomère choisi parmi l'acide acrylique, l'acide méthacrylique, des esters d'acide acrylique et des esters d'acide méthacrylique ; des polyesters de diglycol, cyclohexanediméthanol, acide isophtalique et acide sulfo-isophtalique.

11. Composition selon l'une quelconque des revendications 7 à 10, **caractérisée en ce qu'**en tant que polymère de soin capillaire ou fixant les cheveux est contenu un polymère à groupes cationiques ou pouvant être rendus cationiques, choisi parmi des dérivés cationiques de cellulose à base d'hydroxyéthylcellulose et chlorure de diallyldiméthylammonium ; des dérivés cationiques de cellulose à base d'hydroxyéthylcellulose et d'époxyde substitué par triméthylammonium ; le poly(chlorure de diméthyldiallylammonium) ; des copolymères d'acrylamide et chlorure de diméthyldiallylammonium ; des polymères d'ammonium quaternaire formés par la réaction de sulfate de diéthyle et d'un copolymère de vinylpyrrolidone und méthacrylate de diméthylaminoéthyle ; des polymères d'ammonium quaternaire à base de chlorure de méthylvinylimidazolium et vinylpyrrolidone ; le Polyquaternium-35 ; un polymère de chlorure de triméthylammonium-méthacrylate d'éthyle ; le Polyquaternium-57 ; des diméthylpolysiloxanes substitués en bout de chaîne par des groupes ammonium quaternaires ; un copolymère de vinylpyrrolidone, diméthylaminopropylméthacrylamide et chlorure de méthacryloylaminopropyllauryldiméthylammonium ; le chitosane et ses sels ; des hydroxyalkylchitosanes et leurs sels ; des alkyl-hydroxyalkylchitosanes et leurs sels ; des éthers alkyliques de N-hydroxyalkylchitosane ; un copolymère de vinylcaprolactame, vinylpyrrolidone et méthacrylate de diméthylaminoéthyle ; des copolymères de vinylpyrrolidone et méthacrylate de diméthylaminoéthyle ; des copolymères de vinylpyrrolidone, vinylcaprolactame et diméthylaminopropylacrylamide ; des poly- ou oligoesters formés à partir d'au moins un premier type de monomère qui est choisi parmi des acides hydroxylés substitués par au moins un groupe ammonium quaternaire.

12. Composition selon l'une quelconque des revendications 7 à 11, **caractérisée en ce qu'**en tant que polymère de soin capillaire ou fixant les cheveux est contenu un polymère zwitterionique et/ou un polymère amphotère, choisi parmi des copolymères d'octylacrylamide, acide acrylique, méthacrylate de butylaminoéthyle, méthacrylate de méthyle et méthacrylate d'hydroxypropyle ; des copolymères d'acrylate de lauryle, acrylate de stéaryle, amino-oxyméthacrylate d'éthyle et d'au moins un monomère choisi parmi l'acide acrylique, l'acide méthacrylique, des esters d'acide acrylique et des esters d'acide méthacrylique ; des copolymères de méthacryloyléthylbtaïne et d'au moins un monomère choisi parmi l'acide méthacrylique et des esters d'acide méthacrylique ; des copolymères d'acide acrylique, acrylate de méthyle et chlorure de méthacrylamidopropyltriméthylammonium ; des oligomères ou polymères pouvant être préparés à partir de crotonobétaïnes ou d'esters de crotonobétaïnes quaternaires.

13. Composition selon l'une quelconque des revendications 7 à 12, **caractérisée en ce qu'**en tant que polymère de soin capillaire ou fixant les cheveux est contenu un polymère non ionique choisi parmi la polyvinylpyrrolidone, le polyvinylcaprolactame, des copolymères vinylpyrrolidone/acétate de vinyle, le poly(alcool vinylique), un copolymère isobutylène/éthylmaléimide/hydroxyéthylmaléimide ; des copolymères de vinylpyrrolidone, acétate de vinyle et propionate de vinyle.

14. Composition selon l'une quelconque des revendications 7 à 13, **caractérisée en ce qu'**en tant que composé silicone de soin capillaire est contenu au moins un composé silicone choisi parmi des diméthylsiloxanes cycliques, des polydiméthylsiloxanes linéaires ; des polymères séquences à base de polydiméthylsiloxane et polyoxyéthylène et/ou polyoxypropylène, des polydiméthylsiloxanes à radicaux polyoxyéthylène ou polyoxypropylène latéraux ou en bout de chaîne, des polydiméthylsiloxanes à groupes hydroxy en bout de chaîne, des polydiméthylsiloxanes substitués par phényle, des émulsions de silicone, des élastomères silicone, des cires de silicone, des gommes de silicone, des silicones substitués par amino et des silicones substitués par un ou plusieurs groupes ammonium quaternaire.

15. Composition selon l'une quelconque des revendications précédentes concernant une composition, **caractérisée en ce que** la substance photoprotectrice est choisie parmi le 4-méthoxycinnamate de 2-éthylhexyle, le méthoxycinnamate de méthyle, l'acide 2-hydroxy-4-méthoxy-benzophénone-5-sulfonique et des p-aminobenzoates polyéthoxylés.

16. Composition selon l'une quelconque des revendications précédentes concernant une composition, **caractérisée en ce qu'**en tant qu'huile de soin capillaire ou en tant que cire de soin capillaire ou fixant les cheveux est contenue une huile ou une cire choisies parmi des cires de paraffine, des cires polyoléfiniques, la lanoline, les alcolanums, la cire de candelilla, la cire d'olive, la cire de carnauba, la cire du Japon, la cire de pomme, des graisses durcies, des esters d'acides gras, des glycérides d'acides gras, des triglycérides d'acides gras, des cires de polyéthylèneglycol, des cires de silicone, des huiles de silicone, des huiles minérales, des huiles isoparaffiniques, des huiles de paraffine, le squalane, l'huile de tournesol, l'huile de coprah, l'huile de ricin, l'huile de lanoline, l'huile de jojoba, l'huile de maïs, l'huile de soja.

17. Composition selon l'une quelconque des revendications précédentes concernant une composition, **caractérisée en ce que** la substance active (B) de soin capillaire, protégeant les cheveux ou réparant les cheveux est choisie parmi la bétaïne ; le panthénol ; le panthényléthyléther ; le sorbitol ; des hydrolysats de protéines ; des extraits de plantes ; des copolymères séquencés A-B d'acrylates d'alkyle et de méthacrylates d'alkyle ; des copolymères séquencés A-B de méthacrylates d'alkyle et acrylonitrile ; des copolymères séquencés A-B-A de lactide et oxyde d'éthylène ; des copolymères séquencés A-B-A de caprolactone et oxyde d'éthylène ; des copolymères séquencés A-B-C de composés alkylène ou alkadiène, styrène et méthacrylates d'alkyle ; des copolymères séquencés A-B-C d'acide acrylique, styrène et méthacrylate d'alkyle ; des copolymères séquencés en étoile ; des polymères hyper-ramifiés, des dendrimères, des 3,4-polyéthylènedioxythiophènes intrinsèquement conducteurs de l'électricité et des polyanilines intrinsèquement conductrices de l'électricité.

18. Composition selon l'une quelconque des revendications précédentes concernant une composition, **caractérisée en ce qu'**est contenu au moins un tensioactif de soin capillaire, choisi parmi des produits de fixation par addition de 2 à 30 moles d'oxyde d'éthylène et/ou de 1 à 5 moles d'oxyde de propylène sur des alcools gras en C₈-C₂₂, des produits de fixation par addition de 2 à 30 moles d'oxyde d'éthylène et/ou de 1 à 5 moles d'oxyde de propylène sur des acides gras en C₁₂-C₂₂, des produits de fixation par addition de 2 à 30 moles d'oxyde d'éthylène et/ou de 1 à 5 moles d'oxyde de propylène sur des alkylphénols ayant de 8 à 15 atomes de carbone dans le groupe alkyle, des mono- et diesters d'acides gras en C₁₂-C₂₂ de produits de fixation par addition de 1 à 30 moles d'oxyde d'éthylène sur le glycérol, des produits de fixation par addition de 5 à 60 moles d'oxyde d'éthylène sur l'huile de ricin ou sur l'huile de ricin durcie (hydrogénée), des mono-, di- et/ou triesters de l'acide phosphorique avec des produits de fixation par addition de 2 à 30 moles d'oxyde d'éthylène sur des alcools gras en C₈-C₂₂, des esters de saccharose et d'un ou deux acides gras en C₈-C₂₂, des esters de sorbitanne et d'un, de deux ou trois acides gras en C₈-C₂₂ et ayant un degré d'éthoxylation de 4 à 20, des esters d'acides gras et de polyglycéryle constitués d'un, de deux ou plus de deux acides gras en C₈-C₂₂ et de polyglycérol comportant 2 à 20 motifs glycéryle, des alkylglycosides, des composés alkyl(C₈-C₂₂)-diméthylbenzylammonium, des composés alkyl(C₈-C₂₂)-triméthylammonium, des composés alkyl(C₈-C₂₂)-diméthylhydroxyéthylammonium, des composés di[alkyl(C₈-C₂₂)]-dimethylammonium, des sels d'alkyl(C₈-C₂₂)-pyridinium, des éthersulfates d'alkyl(C₈-C₂₂)amidoéthyltriméthylammonium, des oxydes d'alkyl(C₈-C₂₂)méthylamines, des oxydes d'alkyl(C₈-C₂₂)aminoéthyldiméthylamines, des amido-amines et des amido-amines rendues quaternaires.

19. Composition selon l'une quelconque des revendications 7 à 18, **caractérisée en ce qu'**elle se trouve sous forme d'un gel capillaire, sous forme d'une lotion capillaire visqueuse ou sous forme d'un gel capillaire à pulvériser qui est pulvérisé au moyen d'un dispositif mécanique, en outre contient au moins un polymère épaississant et présente une viscosité d'au moins 250 mPa.s (mesurée à l'aide d'un viscosimètre Bohlin Rheometer CS, élément de mesure C25, à 25°C et à une vitesse de cisaillement de 50 s⁻¹).

20. Composition selon l'une quelconque des revendications 7 à 18, **caractérisée en ce qu'**elle se trouve sous forme d'une émulsion H/E, d'une émulsion E/H ou d'une microémulsion et contient au moins une huile ou cire ainsi qu'au moins un émulsifiant.

21. Composition selon l'une quelconque des revendications 7 à 18, sous forme d'une laque pour cheveux, **caractérisée en ce qu'**elle se trouve soit en association avec un pulvérisateur mécanique à pompe, soit en association avec au moins un propulseur choisi parmi le propane, le butane, l'éther diméthylique et des hydrocarbures fluorés.

22. Composition selon l'une quelconque des revendications 7 à 18, **caractérisée en ce qu'**elle se trouve sous forme d'un produit pouvant être transformé en mousse, en association avec un dispositif pour la transformation en mousse, contient au moins un tensioactif moussant ou au moins un polymère moussant et soit en association avec un dispositif mécanique de moussage à pompe, soit en association avec au moins un propulseur choisi parmi le propane, le butane, l'éther diméthylique et des hydrocarbures fluorés.

23. Composition selon l'une quelconque des revendications 7 à 18, **caractérisée en ce qu'**elle se trouve sous forme d'une cire capillaire solide et contient au moins une cire mentionnée dans la revendication 17.

24. Composition selon l'une quelconque des revendications 7 à 18, **caractérisée en ce qu'**elle se trouve sous forme d'un produit de nettoyage pour cheveux et contient de 0,01 à 40 % en poids d'au moins un tensioactif anionique amphotère ou non ionique nettoyant les cheveux et de 50 à 90 % en poids d'eau.

25. Composition selon l'une quelconque des revendications 7 à 18, **caractérisée en ce qu'**elle est une composition de mise en forme permanente et contient au moins une substance réduisant la kératine, choisie parmi des composés mercapto réduisant la kératine.

26. Composition selon l'une quelconque des revendications 7 à 18, **caractérisée en ce qu'**elle est une composition de teinture des cheveux en blond, une composition de teinture des cheveux par oxydation ou un fixateur d'ondulation permanente, ayant une teneur en au moins un oxydant.

27. Composition selon l'une quelconque des revendications 7 à 18, **caractérisée en ce qu'**elle est une composition de nuançage pour cheveux ou une composition de teinture des cheveux par oxydation et contient au moins un colorant naturel et/ou synthétique pour cheveux s'épuisant directement sur le cheveu ou au moins un précurseur de colorant d'oxydation.

28. Procédé pour le traitement des cheveux, dans lequel
- on prépare une composition de traitement des cheveux selon l'une quelconque des revendications 7 à 27,
- on applique sur les cheveux la composition de traitement des cheveux et
- soit on élimine des cheveux la composition de traitement des cheveux par rinçage après un temps d'action, soit on la laisse sur les cheveux.
